# FASCICULE DE BREVET EUROPEEN

(11) **EP 4 003 271 B1**
(45) Date de publication et mention de la délivrance du brevet: **31.05.2023**
(21) Numéro de dépôt: 20754331.5
(22) Date de dépôt: 21.07.2020
(51) Int. Cl.: A61K 8/02, A61K 8/25, A61K 8/34, A61Q 1/12, A61K 8/81

(54) **COMPOSITION COSMÉTIQUE SOUS LA FORME D'UNE POUDRE COMPACTE, RÉSISTANTE AUX CHOCS**
KOSMETISCHE ZUSAMMENSETZUNG IN FORM EINES KOMPAKTEN SCHLAGFESTEN PULVERS
COSMETIC COMPOSITION IN THE FORM OF A COMPACT, IMPACT-RESISTANT POWDER

(30) Priorité: 31.07.2019 FR 1908744
(43) Date de publication de la demande: 01.06.2022
(73) Titulaire: Société d'Exploitation de Produits pour les Industries Chimiques SEPPIC, 75321 Paris cedex 07 (FR); Strand Cosmetics Europe, 69210 Lentilly (FR)
(72) Inventeur: CAMBOS, Sophie, 75321 PARIS CEDEX 07 (FR); TAILLEBOIS, Cécile, 75321 PARIS CEDEX 07 (FR); MSIKA, Philippe, 75321 PARIS CEDEX 07 (FR); COUVAL, Emmanuelle, 69210 LENTILLY (FR); VERDELET, Isabelle, 69210 LENTILLY (FR); RISSELADA, Christa, 69210 LENTILLY (FR); PASQUET, Julia, 69210 LENTILLY (FR)
(74) Mandataire: Air Liquide
(86) Numéro de dépôt international: PCT/FR2020/051313
(87) Numéro de publication internationale: WO 2021/019151

(56) Documents cités:
- FR-A1- 2 980 108
- US-A1- 2016 167 040

## Description

La présente invention concerne une composition comprenant au moins un agent de charge, au moins un pigment coloré, et au moins un polymère de type polyélectrolyte anionique réticulé, ladite composition se présentant sous une forme pulvérulente à température ambiante.

La présente invention concerne également une composition cosmétique à usage topique se présentant sous une forme compacte comprenant ladite composition pulvérulente et au moins un agent liant, présentant une résistance améliorée aux chocs.

La présente invention concerne aussi l'utilisation de telles compositions cosmétiques compactes comme produits de maquillage de la peau humaine, et un procédé de maquillage de la peau humaine mettant en oeuvre de telles compositions compactes.

Certaines compositions cosmétiques à usage topique destinées à maquiller la peau, comme par exemple les fonds de teints pour le visage, les poudres de teint pour le visage, les fards à paupières, les fards à joues, se présentent généralement sous la forme de poudres compactes ou coulées.

Par « poudres compactes », on entend au sens de la présente invention des compositions constituées principalement par un mélange de poudres, comprenant au moins un agent de charge, au moins un pigment, au moins un polymère de type polyélectrolyte anionique réticulé, et au moins un agent liant. Une fois constitué, ce mélange est mis en forme par compression dans un godet.

Ces poudres compactes sont généralement utilisées par prélèvement d'une petite quantité puis par application sur la peau à l'aide des doigts, ou d'un applicateur comme par exemple un pinceau ou une éponge.

Pour disposer d'une poudre compacte de qualité, il est nécessaire qu'elle soit facilement préhensible, qu'elle soit facile à étaler, que l'étalement en résultant soit uniforme, qu'elle présente un toucher doux une fois étalée sur la peau. Plus spécifiquement, une poudre compacte de qualité doit présenter une surface lisse, plane et conserver ses propriétés dans le temps. Elle doit également présenter une résistance aux chocs suffisamment importante pour ne pas être fragmentée et conserver ses propriétés.

Pour améliorer les propriétés de résistance aux chocs, les concepteurs de poudres compactes destinées au maquillage de la peau ont mis au point des solutions reposant sur l'optimisation d'agents liants spécifiques.

Ainsi la demande internationale de brevet publiée sous le numéro WO9/17660A1 décrit l'utilisation d'un mélange d'au moins une huile de silicone, d'au moins une cire de silicone, et d'au moins une résine de silicone, constituant un liant gras destiné à être mélangé avec une phase particulaire solide, pour ultérieurement atteindre une poudre compacte. Cependant, les quantités d'agents liants à employer s'avèrent importantes et l'aspect de la surface de la poudre compacte devient alors grasse et le transfert sur le doigt ou l'applicateur avant application est moins efficace.

La demande de brevet européen publiée sous le numéro EP 0 171 979 A2 décrit un procédé de préparation d'une composition se présentant sous la forme d'une poudre compacte stable au stockage, résistante aux chocs, caractérisée par une surface plane et lisse. Le procédé objet de la demande de brevet européen EP 0 717 979 A2 comprend une étape de dispersion de la phase pulvérulente dans une émulsion huile-dans-eau préalablement formée, puis une étape de coulée de la dispersion obtenue dans un moule, puis une étape de séchage par lyophilisation de la dispersion précédemment obtenue. Un tel procédé présente cependant l'inconvénient d'être mis en oeuvre avec difficulté à l'échelle industrielle car il fait intervenir des équipements coûteux et très consommateurs d'énergie.

La demande de brevet américain publiée sous le numéro US 2005/0186235A1 décrit des compositions cosmétiques se présentant sous la forme de poudres compactes et comprenant une phase pulvérulente, une phase grasse solide, et nécessitant pour sa préparation la mise en oeuvre d'une étape de fusion de la phase grasse solide (ou cire) qui s'avère très consommatrice d'énergie.

La demande de brevet américain publiée sous le numéro US 2016/167040A1 décrit un polymère de type polyélectrolyte anionique réticulé dont le squelette polymérique est constitué du sel d'ammonium du 2-methyl-2-[(1-oxo-2-propenyl)amino]-1-propanesulfonate (ATBS), du (2-hydroxyethyl) acrylate (BEA), du méthacrylate de stéaryle (SMA) et de méthacrylate de lauryle (LAUMA), réticulé avec du trimethylolpropane triacrylate (ATBS/HEA/SMA/LAUMA: 88.1/9.9/1.5/0.5; Polyélectrolyte 1). La demande de brevet américain publiée sous le numéro US 2016/167040A1 décrit également une composition cosmétique de poudre compacte (exemple 52) qui comprend 2% massique du Polyélectrolyte 1.

La demande de brevet Français publiée sous le numéro FR2980108A1 décrit des compositions de maquillage sous forme de poudres compactes résistantes aux chocs, comprenant une phase pulvérulente et une phase liquide, mais dénuée de polymère de type polyélectrolyte anionique réticulé.

Pour tenter de s'affranchir de ces inconvénients, les inventeurs ont cherché à disposer d'une composition cosmétique se présentant sous la forme d'une poudre compacte, résistante aux chocs, se caractérisant par une bonne qualité de transfert pour favoriser la préhension et l'application sur la peau, et préparée selon un procédé ne nécessitant pas l'apport de quantités importante d'énergie.

Ainsi, la présente invention a pour objet une composition pulvérulente (C₁) sous forme de poudre comprenant pour 100% de sa masse :
i) de 84,3% à 95,8% massique, de préférence de 86,9% à 95,2% massique, et encore plus préférentiellement de 89,5% à 94,65% massique d'au moins un agent de charge (AC)
ii) de 4% à 15% massique, de préférence de 4.5% à 12.5% massique, et encore plus préférentiellement de 5% à 10% massique d'au moins un pigment coloré (PC)
iii) de 0,2% à 0,7% massique, de préférence de 0.3% à 0.6 % massique, et encore plus préférentiellement de 0.35 % à 0.5% massique d'au moins un polymère de type polyélectrolyte anionique réticulé (P) qui comporte pour 100 % molaire, de 65% molaire à 95 % molaire d'unités monomériques issues de l'acide 2-méthyl 2-[(1-oxo 2-propènyl) amino] 1-propanesulfonique partiellement ou totalement salifié, de 4,8 % molaire à 25 % molaire d'unités monomériques issues d'au moins un monomère neutre choisi parmi les éléments du groupe constitué par le 2-hydroxy éthyl acrylate, le 2-hydroxy éthyl méthacrylate, les N,N-dialkyl acrylamides dans lesquels chacun des groupes alkyle comportent entre un et quatre atomes de carbones, et de 0,1 à 5 % molaire d'unités monomériques issues d'au moins un monomère de formule (I) : 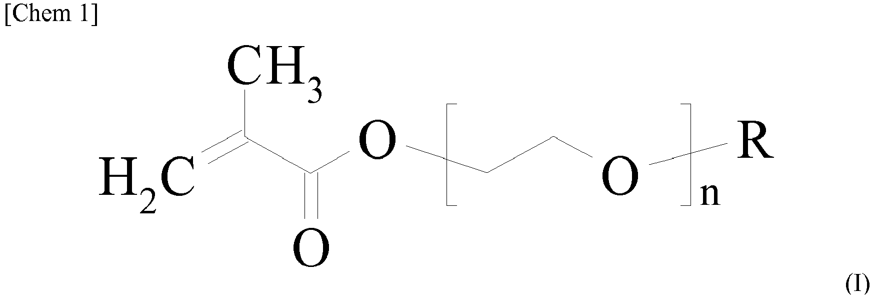 dans laquelle R représente un radical alkyle linéaire ou ramifié comportant de huit à vingt atomes de carbone et n représente un nombre supérieur ou égal à zéro et inférieur ou égal à vingt, et de 0,1 % à 5 % molaire d'unités monomériques issues d'au moins un monomère de réticulation diéthylénique ou polyéthylénique.

Par « agent de charge », on entend au sens de la présente invention, une substance chimique solide, non miscible à l'eau, et dispersée dans une matrice. Un agent de charge est introduit dans un mélange, en l'occurrence une composition de maquillage, pour en améliorer le coût, ainsi que certaines propriétés comme par exemple le pouvoir couvrant sur la peau, la densité ou la masse volumique de la composition pulvérulente.

Selon un aspect plus particulier, la composition pulvérulente (C1) comprend au moins 90% en volume de particules de diamètre inférieur ou égal à 200 micromètres, et plus particulièrement de diamètre inférieur ou égal à 100 micromètres. On signifie dans le cadre de la présente invention, que la poudre de composition C1 est assimilée à une poudre de particules assimilées à des sphères dont le diamètre est inférieur ou égal à 200 micromètres, et plus particulièrement dont le diamètre est inférieur ou égal à 100 micromètres. Notons que cette taille de particules permet d'atteindre une meilleure cohésion de la poudre compacte ultérieurement obtenue.

La détermination de ce paramètre est réalisée au moyen d'un analyseur à diffraction laser, par exemple le "granulomètre laser MALVERN Mastersizer^{™} 2000, équipé d'un dispositif de dispersion, par exemple le dispositif de dispersion de type MS1-Small Volume Sample Dispersion^{™}, et relié à un logiciel de calcul, qui permet d'obtenir un diffractogramme consistant en une superposition des images de diffraction de chaque taille de particules représentées dans la poudre analysée.

Dans l'analyse des données ainsi recueillies, une distribution de taille initiale est estimée et le diffractogramme théorique est calculé, puis comparé avec les données réelles enregistrées. Les différences entre les données estimées et les données réelles sont ensuite minimisées en utilisant la méthode des moindres carrées. Le logiciel calcule ensuite la distribution en volume en tant que résultat fondamental et toute autre information est déduite de ce résultat en supposant que les particules ont une forme sphérique.

Cette méthode de détermination est particulièrement bien adaptée à la caractérisation des poudres dont les particules qui la constituent sont assimilées à des sphères de diamètres compris entre 3000 micromètres et 0,1 micromètres, et pour des poudres sèches. L'utilisation de ce type de méthode a particulièrement montré de bons résultats pour des tailles de particules supérieures à 10 micromètres [P. Bowen, "Particle Size Distribution Measurement from Millimeters to Nanometers and from Rods to Platelets"; J. Dispersion Science and Technology, 23(5), pp. 631-662 (2002)].

Par polymère de type polyélectrolyte anionique réticulé (P), on désigne, dans la définition de la composition pulvérulente (C₁) un polyélectrolyte anionique réticulé non linéaire se présentant à l'état de réseau tridimensionnel insoluble dans l'eau, mais gonflable à l'eau et conduisant donc à l'obtention d'un gel chimique.

Par partiellement salifié ou totalement salifié, on signifie, dans la définition de la composition pulvérulente (C₁), que la fonction acide de l'acide 2-méthyl 2-[(1-oxo 2-propènyl) amino] 1-propanesulfonique est partiellement ou totalement salifiée, généralement sous forme de sel de métal alcalin, comme par exemple le sel de sodium ou le sel de potassium, ou sous forme de sel d'ammonium.

Dans la composition pulvérulente (C₁) telle que définie ci-dessus et objet de la présente invention, ledit polymère de type polyélectrolyte anionique réticulé (P) mis en oeuvre comporte, pour 100% molaire, entre 65% molaire et 95% molaire, plus particulièrement entre 65% molaire et 90% molaire, et encore plus particulièrement entre 68% molaire et 95% molaire, et encore plus particulièrement entre 70% molaire et 95% molaire, et encore plus particulièrement entre 70% molaire et 90% molaire d'unités monomériques issues de l'acide 2-méthyl 2-[(1-oxo 2-propènyl) amino] 1-propanesulfonique partiellement ou totalement salifié.

Dans la composition pulvérulente (C₁) telle que définie ci-dessus et objet de la présente invention, ledit polymère de type polyélectrolyte anionique réticulé (P) mis en oeuvre comporte, pour 100% molaire, entre 4,8% molaire et 25% molaire, plus particulièrement entre 4,8% et 20% molaire, et encore plus particulièrement entre 5% molaire et 20% molaire d'unités monomériques d'au moins un monomère neutre choisi parmi les éléments du groupe constitué par le 2-hydroxyéthyl acrylate, le 2-hydroxyéthyl méthacrylate, les N,N-dialkyl acrylamides dans lesquels chacun des groupes alkyle comportent entre un et quatre atomes de carbone.

Selon un aspect particulier, dans le polymère de type polyélectrolyte anionique réticulé (P) mis en oeuvre dans la composition pulvérulente (C₁) objet de la présente invention, le monomère neutre choisi parmi les N,N-dialkyl acrylamides, dans lesquels chacun des groupes alkyles comportent entre un et quatre atomes de carbone, est choisi parmi le N,N-diméthyl acrylamide, le N,N-diéthyl acrylamide, le N,N-dipropyl acrylamide.

Dans la composition pulvérulente (C₁) telle que définie ci-dessus et objet de la présente invention, ledit polymère de type polyélectrolyte anionique réticulé (P) mis en oeuvre comporte, pour 100% molaire, entre 0,1% molaire et 5% molaire, encore plus particulièrement entre 0,1% et 4,0% molaire d'unités monomériques d'au moins un monomère de formule (I).

Dans la formule (I) du monomère présent dans ledit polymère de type polyélectrolyte anionique réticulé (P) mis en oeuvre dans la composition pulvérulente (C₁) objet de la présente invention, par radical alkyle linéaire ou ramifié comportant de huit à vingt atomes de carbone, on désigne plus particulièrement pour R :
- ou bien un radical dérivé des alcools primaires linéaires tels que par exemple, le radical octyle, décyle, undécyle, dodécyle, tridécyle, tétradécyle, pentadécyle, hexadécyle, heptadécyle, octadécyle, nonadécyle ou eicosyle ;
- ou bien un radical dérivé des alcools de Guerbet, qui sont des 1-alcanols ramifiés répondant à la formule générale :

   CH₃-(CH₂)ₚ-CH[CH₃-(CH₂)ₚ₋₂]-CH₂OH,

   dans laquelle p représente un nombre entier compris entre 2 et 9, tels que, par exemple, les radicaux 2-éthyl hexyle, 2-propyl heptyle, 2-butyl octyle, 2-pentyl nonyle, 2-hexyl décyle ou 2-octyl dodécyle ;
- ou bien un radical dérivé des isoalcanols répondant à la formule générale :

   CH₃-CH(CH₃)-(CH₂)ₘ-CH₂OH,

   dans laquelle m représente un nombre entier compris entre 2 et 16, tels que, par exemple, les radicaux 4-méthyl pentyle, 5-méthyl hexyle, 6-méthyl heptyle, 15-méthyl pendadécyle ou 16-méthyl heptadécyle, soit les radicaux 2-hexyl octyle, 2-octyl décyle ou 2-hexyl dodécyle. Selon un aspect particulier, l'invention a pour objet une composition pulvérulente (C₁) telle que décrite précédemment, caractérisée en ce que ledit polymère de type polyélectrolyte anionique réticulé (P) comporte pour 100% molaire :
- de 65% molaire à 95% molaire, de préférence de 70% molaire à 95% molaire d'unités monomériques issues de l'acide 2-méthyl 2-[(1-oxo 2-propènyl) amino] 1-propanesulfonique partiellement ou totalement salifié ;
- de 4,8% molaire à 25% molaire, de préférence de 4,8% molaire à 20% molaire d'unités monomériques issues d'un monomère neutre choisi parmi les éléments du groupe constitué par le 2-hydroxy éthyl acrylate, le 2-hydroxy éthyl méthacrylate, les N,N-dialkyl acrylamides dans lesquels chacun des groupes alkyle comportent entre un et quatre atomes de carbone ;
- de 0,1% à 5% molaire, de préférence de 0,5% à 5% molaire, et plus préférentiellement de 0,5% molaire à 4% molaire d'unités monomériques issues d'un monomère de formule (I) telle que définie précédemment,
- de 0,1% à 5% molaire, de préférence de 0,5% à 5% molaire, plus préférentiellement de 0,5% à 3% molaire d'unités monomériques issues d'au moins un monomère de réticulation diéthylénique ou polyéthylénique.

Selon un aspect particulier, l'invention a pour objet une composition pulvérulente (C₁) telle que décrite précédemment, caractérisée en ce que dans le polymère de type polyélectrolyte anionique réticulé (P), ledit monomère neutre est choisi parmi les éléments du groupe constitué par le 2-hydroxyéthyl acrylate ou le N,N diméthyl acrylamide.

Selon un aspect particulier, l'invention a pour objet une composition pulvérulente (C₁) telle que décrite précédemment, caractérisée en ce que dans ledit polymère de type polyélectrolyte anionique réticulé (P) et pour ledit monomère de formule (I) telle que définie précédemment, R représente un radical alkyle comportant de 12 à 18 atomes de carbone.

Selon un autre aspect particulier, l'invention a pour objet une composition pulvérulente (C₁) telle que décrite précédemment, caractérisée en ce que dans ledit polymère de type polyélectrolyte anionique réticulé (P), R représente le radical n-dodécyle ou le radical isododécyle ou le radical n-tétradécyle ou le radical n-hexadécyle ou le radical n-octadécyle et n représente un entier compris entre 3 et 20.

Selon un autre aspect particulier, l'invention a pour objet une composition pulvérulente (C₁) telle que décrite précédemment, caractérisée en ce que dans ledit polymère de type polyélectrolyte anionique réticulé (P), ledit monomère de formule (I) telle que définie précédemment est le méthacrylate de lauryle tétraéthoxylé de formule (I₁) correspondant à la formule (I) pour laquelle n est égal à quatre et R représente le radical n-dodécyle.

Selon un autre aspect particulier, l'invention a pour objet une composition pulvérulente (C₁) telle que décrite précédemment, caractérisée en ce que dans ledit polymère de type polyélectrolyte anionique réticulé (P), ledit monomère de formule (I) telle que définie précédemment est le méthacrylate de stéaryle eicosaéthoxylé de formule (I₄) correspondant à la formule (I) pour laquelle n est égal à vingt et R représente le radical n-octadécyle.

Selon un autre aspect particulier, l'invention a pour objet une composition pulvérulente (C₁) telle que décrite précédemment, caractérisée en ce que dans ledit polymère de type polyélectrolyte anionique réticulé (P), R représente le radical n-dodécyle ou le radical isododécyle ou le radical n-tétradécyle ou le radical n-hexadécyle ou le radical n-octadécyle et n est égal à zéro.

Selon un autre aspect particulier, l'invention a pour objet une composition pulvérulente (C₁) telle que décrite précédemment, caractérisée en ce que dans ledit polymère de type polyélectrolyte anionique réticulé (P), ledit monomère de formule (I) telle que définie précédemment est le méthacrylate de lauryle de formule (I₂) correspondant à la formule (I) dans laquelle R représente un radical n-dodécyle et/ou un radical isododécyle et dans laquelle n est égal à zéro.

Selon un autre aspect particulier, l'invention a pour objet une composition pulvérulente (C₁) telle que décrite précédemment, caractérisée en ce que dans ledit polymère de type polyélectrolyte anionique réticulé (P), ledit monomère de formule (I) telle que définie précédemment est le méthacrylate de stéaryle de formule (I₃) correspondant à la formule (I) dans laquelle R représente un radical n-octadécyle et dans laquelle n est égal à zéro.

Selon un aspect plus particulier, l'invention a pour objet une composition pulvérulente (C₁) telle que décrite précédemment, caractérisée en ce que dans ledit polymère de type polyélectrolyte anionique réticulé (P), ledit monomère de formule (I) est choisi parmi les éléments du groupe constitué par le méthacrylate de lauryle tétraéthoxylé de formule (I₁) correspondant à la formule (I) dans laquelle R représente le radical n-dodécyle et dans laquelle n est égal à quatre, par le méthacrylate de lauryle de formule (I₂), correspondant à la formule (I) dans laquelle R représente un radical n-dodécyle et dans laquelle n est égal à zéro, par le méthacrylate d'isodécyle de formule (I'₂), correspondant à la formule (I) dans laquelle R représente un radical isododécyle et dans laquelle n est égal à zéro, et par le méthacrylate de stéaryle de formule (I₃), correspondant à la formule (I) dans laquelle R représente un radical n-octadécyle et dans laquelle n est égal à zéro.

Selon un aspect particulier, l'invention a pour objet une composition pulvérulente (C₁) telle que décrite précédemment, caractérisée en ce que dans ledit polymère de type polyélectrolyte anionique réticulé (P), ledit monomère de réticulation diéthylénique ou polyéthylénique est choisi parmi le diméthacrylate d'éthylèneglycol, le tétraallyloxyéthane, le diacrylate d'éthylèneglycol, le diallyl urée, le triallylamine, le triméthylol propanetriacrylate, le méthylène-bis(acrylamide) ou un mélange de ces composés.

Le polyélectrolyte anionique réticulé (P) mis en oeuvre dans la composition pulvérulente (C₁) telle que décrite précédemment peut également comprendre divers additifs, tels que des agents complexants, des agents de transfert ou des agents limiteurs de chaîne.

Par agents de transfert ou par agents limiteurs de chaîne, on désigne les composés chimiques qui, lorsque présents lors de la réaction de polymérisation radicalaire de préparation du polyélectrolyte anionique réticulé (P), génèrent des réactions de terminaison, comme des réactions de dismutation ou de recombinaison, entrainant la formation de chaînes polymériques de moindre longueur et donc de moindre poids moléculaire. Comme agents de transfert ou comme agents limiteurs de chaîne, on peut citer les thiols comme le dodécyl mercaptan, les halogénures comme le tétrachlorure de carbone, les alcools courts comme l'éthanol, le n-propanol, l'isopropanol, le n-butanol, l'iso-butanol, l'hypophosphite de sodium, de formule chimique NaPO₂H₂.

Selon un aspect particulier, l'invention a pour objet une composition pulvérulente (C₁) telle que décrite précédemment, caractérisée en ce que ledit polymère de type polyélectrolyte anionique réticulé (P) est choisi parmi les polymères de l'acide 2-méthyl 2-[(1-oxo 2-propènyl) amino] 1-propanesulfonique partiellement salifié sous forme de d'ammonium, du N,N-diméthyl acrylamide et du méthacrylate de lauryle tétraéthoxylé, réticulé au triméthylol propanetriacrylate ou parmi les polymères de l'acide 2-méthyl 2-[(1-oxo 2-propènyl) amino] 1-propanesulfonique partiellement salifié sous forme de sel d'ammonium, du N,N-diméthyl acrylamide et du méthacrylate de stéaryle d'eicosaéthoxylé, réticulé au triméthylol propanetriacrylate.

Selon un aspect encore plus particulier, l'invention a pour objet une composition pulvérulente (C₁) telle que décrite précédemment, caractérisée en ce que ledit polymère de type polyélectrolyte anionique réticulé (P) est un polymère de l'acide 2-méthyl 2-[(1-oxo 2-propènyl) amino] 1-propanesulfonique partiellement ou totalement salifié sous forme de sel d'ammonium, du N,N-diméthyl acrylamide et du méthacrylate de lauryle tétraéthoxylé de formule (I₁), réticulé au triméthylol propanetriacrylate.

Selon un aspect encore plus particulier, l'invention a pour objet une composition pulvérulente (C₁) telle que décrite précédemment, caractérisée en ce que ledit polymère de type polyélectrolyte anionique réticulé (P) comprend pour 100% molaire :
- De 65% molaire à 95% molaire d'unités monomériques issues de l'acide 2-méthyl 2-[(1-oxo 2-propènyl) amino] 1-propanesulfonique salifié sous forme d'ammonium;
- De 4,8% molaire à 25% molaire d'unités monomériques issues du N,N-diméthyl acrylamide,
- De 0,1% à 5% molaire d'unités monomériques issues du méthacrylate de lauryle tétraéthoxylé de formule (I₁)
- de 0,1% à 5% molaire d'unités monomériques issues du triméthylolpropanetriacrylate. Selon un aspect encore plus particulier, l'invention a pour objet une composition pulvérulente (C₁) telle que décrite précédemment, caractérisée en ce que ledit polymère de type polyélectrolyte anionique réticulé (P) est un polymère de l'acide 2-méthyl 2-[(1-oxo 2-propènyl) amino] 1-propanesulfonique partiellement ou totalement salifié sous forme de sel de sodium, du 2-hydroxyéthyl acrylate, du méthacrylate de lauryle de formule (I₂) ou du méthacrylate d'isodécyle de formule (I'₂) et du méthacrylate de stéaryle de formule (I₃) dans un ratio molaire (I₂)/(I₃) ou (I'₂)/(I₃) supérieur ou égal à 1/10 et inférieur ou égal à 10/1, et réticulé au triméthylolpropanetriacrylate.

Selon un aspect encore plus particulier, l'invention a pour objet une composition pulvérulente (C₁) telle que décrite précédemment, caractérisée en ce que ledit polymère de type polyélectrolyte anionique réticulé (P) comprend pour 100% molaire :
- De 70% molaire à 95% molaire d'unités monomériques issues de l'acide 2-méthyl 2-[(1-oxo 2-propènyl) amino] 1-propanesulfonique salifié sous forme de sodium ;
- De 4,8% molaire à 20% molaire d'unités monomériques issues du 2-hydroxy éthyl acrylate,
- de 0,1% molaire à 5% molaire d'unités monomériques issues du méthacrylate de lauryle de formule (I₂) ou du méthacrylate d'isodécyle de formule (I'₂), et du méthacrylate de stéaryle de formule (I₃), dans un ratio molaire (I₂)/(I₃) ou (I'₂)/(I₃) supérieur ou égal à 1/6 et inférieur ou égal à 6/1,
- de 0,1% molaire à 5% molaire de triméthylolpropanetriacrylate.

Le polymère de type polyélectrolyte anionique réticulé (P) peut être obtenu par la mise en oeuvre d'un procédé comprenant :
- Une étape a) au cours de laquelle on mélange l'ensemble des monomères, un agent de neutralisation ammoniaqué de l'acide 2-méthyl 2-[(1-oxo 2-propènyl) amino] 1-propanesulfonique, l'agent réticulant et si nécessaire ou si désiré, les autres additifs dans un solvant organique, de préférence le tert-butanol ;
- Une étape b) au cours de laquelle la réaction de polymérisation est amorcée par introduction d'un initiateur de radicaux libres dans la dispersion préparée à l'étape a), puis est laissée se dérouler jusqu'à sa complétude ;
- Optionnellement une étape c) d'échange de l'ion ammonium présent par l'ion sodium ou l'ion potassium ;
- Une étape d) de précipitation du polymère formé à l'issue de l'étape b), optionnellement de l'étape c) ;
- Optionnellement une étape e) de séchage du précipité obtenu à l'issue de l'étape d). Comme agent de neutralisation ammoniaqué mis en oeuvre à l'étape a) du procédé tel que défini ci-dessus, il y a par exemple l'ammoniac ou encore l'hydrogénocarbonate d'ammonium. A l'étape b) du procédé tel que défini ci-dessus, la réaction de polymérisation est amorcée à une température généralement égale ou supérieure à 50°C à l'aide d'un amorceur radicalaire produisant des radicaux par homolyse, tel que le peroxyde de dilauroyle, l'azobis(isobutyronitrile) ou les dérivés azoïques.

Selon une autre mise en oeuvre du procédé, tel que défini précédemment, la réaction de polymérisation est amorcée par un couple oxydo-réducteur.

A l'étape c) du procédé tel que défini ci-dessus, l'échange du cation ammonium par le cation sodium ou par le cation potassium est optionnellement effectuée avec du ter-butylate de sodium ou du ter-butylate de potassium.

A l'étape d) du procédé tel que défini ci-dessus, la précipitation du polyélectrolyte anionique réticulé (P) est effectuée soit par évaporation du solvant, soit par filtration du précipité.

Le polyélectrolyte anionique réticulé (P) ainsi obtenu est ensuite introduit dans un broyeur, comme par exemple un broyeur à couteaux, en présence d'au moins un pigment coloré, d'au moins un agent de charge, de sorte à obtenir un mélange de poudres broyées.

Ce mélange pulvérulent est introduit dans un godet métallique en présence d'au moins un agent liant, puis soumis à une étape de compactage par l'intermédiaire d'une machine sous pression adaptée.

Selon un aspect particulier, l'invention a pour objet une composition pulvérulente (C₁) telle que décrite précédemment, caractérisée en ce que l'agent de charge (AC) est choisi parmi les éléments du groupe constitué par les charges de type lamellaire minéral, les charges de type lamellaire organique, les charges de type sphérique minéral et les charges de type sphérique organique.

Chaque type de charges permet d'apporter des qualités particulières et différentes à la composition (C₁) selon l'invention. Ainsi, par exemple, les charges de type lamellaire minéral apportent généralement de la douceur, les charges de type sphérique minéral apportent généralement un bon délitage et les charges sphérique organique ont généralement un rôle structurant et apportent de la douceur.

Selon un aspect particulier, l'invention a pour objet une composition pulvérulente (C₁) telle que décrite précédemment, caractérisée en ce que l'agent de charge (AC) est une charge de type lamellaire minéral sélectionnée parmi les éléments du groupe constitué par les talcs ou silicates de magnésium hydratés ; les micas ou aluminosilicates, comme par exemple la muscovite, la margarite, la roscoelite, la lipidolite, la biotite ; les argiles comme par exemple les sericites ; le kaolin ou silicate d'aluminium hydrate ; les nitrures de bore.

Selon un aspect particulier, l'invention a pour objet une composition pulvérulente (C₁) telle que décrite précédemment, caractérisée en ce que l'agent de charge (AC) est une charge de type lamellaire organique sélectionnée parmi les éléments du groupe constitué par les poudre de polymères de tetrafluoroéthylène, la lauroyl lysine.

Selon un aspect particulier, l'invention a pour objet une composition pulvérulente (C₁) telle que décrite précédemment, caractérisée en ce que l'agent de charge (AC) est une charge de type sphérique minéral sélectionnée parmi les éléments du groupe constitué par les oxydes de zinc, les oxydes de titane, le carbonate de calcium, le carbonate de magnésium, l'hydrocarbonate de magnésium, la silice sphérique non poreuse, l'hydroxyapatite, les microsphères de silice à porosité ouverte, les microsphères de silice creuses, les microcapsules de verre, les microcapsules de céramique.

Selon un aspect particulier, l'invention a pour objet une composition pulvérulente (C₁) telle que décrite précédemment, caractérisée en ce que l'agent de charge (AC) est une charge de type sphérique organique sélectionnée parmi les éléments du groupe constitué par les savons métalliques dérivés d'acides organiques carboxyliques ayant de 8 à 22 atomes de carbone, comme par exemple le stéarate de zinc, le stéarate de magnésium, le stéarate de lithium, le laurate de zinc, le myristate de magnésium ; les poudres de polymères synthétiques non expansées, comme par exemple les poudres de polyéthylène, les poudres de polyesters, comme par exemple les poudres d'isophtalate de polyéthylène ou les poudres de téréphtalate de polyéthylène, les poudres de polyamides ; les poudres de polymères synthétiques, réticulés ou non, comme par exemple les poudres d'acide polyacrylique, les poudres d'acide polyméthacrylique, les poudres de polystyrène réticulé par le divinylbenzene, les poudres de résine de silicone ; les poudres d'amidon de maïs, d'amidon de blé, d'amidon de tapioca, d'amidon de riz ; les microsphères de polymères acryliques, les microsphères de polyméthacrylate de méthyle, les microsphères de copolymères de styrène-divinylbenzene. Selon un aspect plus particulier, l'invention a pour objet une composition pulvérulente (C₁) telle que décrite précédemment, caractérisée en ce que l'agent de charge (AC) est choisi parmi les éléments du groupe constitué par :
- Les charges de type lamellaire minéral sélectionnées dans le groupe constitué par les talcs ou silicates de magnésium hydratés ; les micas ou aluminosilicates, comme par exemple la muscovite, la margarite, la roscoelite, la lipidolite, la biotite ; les argiles comme par exemple les sericites ; le kaolin ou silicate d'aluminium hydrate ; les nitrures de bore,
- les charges de type lamellaire organique, comme par exemple les poudre de polymères de tetrafluoroéthylène, la lauroyl lysine,
- les charges de type sphérique minéral sélectionnées parmi les éléments du groupe constitué par les oxydes de zinc, les oxydes de titane, le carbonate de calcium, le carbonate de magnésium, l'hydrocarbonate de magnésium, la silice sphérique non poreuse, l'hydroxyapatite, les microsphères de silice à porosité ouverte, les microsphères de silice creuses, les microcapsules de verre, les microcapsules de céramique,
- les charges de type sphérique organique sélectionnées parmi les éléments du groupe constitué par les savons métalliques dérivés d'acides organiques carboxyliques ayant de 8 à 22 atomes de carbone, comme par exemple le stéarate de zinc, le stéarate de magnésium, le stéarate de lithium, le laurate de zinc, le myristate de magnésium ; les poudres de polymères synthétiques non expansées, comme par exemple les poudres de polyéthylène, les poudres de polyesters, comme par exemple les poudres d'isophtalate de polyéthylène ou les poudres de téréphtalate de polyéthylène, les poudres de polyamides ; les poudres de polymères synthétiques, réticulés ou non, comme par exemple les poudres d'acide polyacrylique, les poudres d'acide polyméthacrylique, les poudres de polystyrène réticulé par le divinylbenzene, les poudres de résine de silicone ; les poudres d'amidon de maïs, d'amidon de blé, d'amidon de tapioca, d'amidon de riz ; les microsphères de polymères acryliques, les microsphères de polyméthacrylate de méthyle, les microsphères de copolymères de styrène-divinylbenzene. Selon un aspect particulier, l'invention a pour objet une composition pulvérulente (C₁) telle que décrite précédemment, caractérisée en ce que le pigment coloré (PC) est choisi parmi les éléments du groupe constitué par, les pigments minéraux, les pigments organiques et les pigments nacrés.

Selon un aspect particulier, l'invention a pour objet une composition pulvérulente (C₁) telle que décrite précédemment, caractérisée en ce que le pigment coloré (PC) est un pigment minéral choisi parmi les éléments du groupe constitué par le dioxyde de titane (rutile ou anatase), éventuellement traité en surface; les oxydes de fer noir, jaune, rouge et brun ; le violet de manganèse ; le bleu outremer ; l'oxyde de chrome éventuellement hydraté; le bleu ferrique.

Selon un aspect particulier, l'invention a pour objet une composition pulvérulente (C₁) telle que décrite précédemment, caractérisée en ce que le pigment coloré (PC) est un pigment organique sélectionné parmi les éléments du groupe constitué par le pigment D&C red, le pigment D&C orange, le pigment D&C yellow, le noir de carbone, les laques à base de carmin de cochenille.

Selon un aspect particulier, l'invention a pour objet une composition pulvérulente (C₁) telle que décrite précédemment, caractérisée en ce que le pigment coloré (PC) est un pigment nacré sélectionné parmi les éléments du groupe constitué par les pigments nacrés blancs, comme par exemple le mica-titane avec des oxydes de fer, le mica-titane avec du bleu ferrique ou de l'oxyde de chrome, le mica-titane avec un pigment organique, les pigments à base d'oxychlorure de bismuth.

Selon un aspect plus particulier, l'invention a pour objet une composition pulvérulente (C₁) telle que décrite précédemment, caractérisée en ce que le pigment coloré (PC) est choisi parmi les éléments du groupe constitué par :
- les pigments minéraux sélectionnés parmi les éléments du groupe constitué par le dioxyde de titane (rutile ou anatase), éventuellement traité en surface; les oxydes de fer noir, jaune, rouge et brun ; le violet de manganèse ; le bleu outremer ; l'oxyde de chrome éventuellement hydraté; le bleu ferrique,
- les pigments organiques sélectionnés parmi les éléments du groupe constitué par le pigment D&C red, le pigment D&C orange, le pigment D&C yellow, le noir de carbone, les laques à base de carmin de cochenille,
- les pigments nacrés sélectionnés parmi les éléments du groupe constitué par les pigments nacrés blancs, comme par exemple le mica-titane avec des oxydes de fer, le mica-titane avec du bleu ferrique ou de l'oxyde de chrome, le mica-titane avec un pigment organique, les pigments à base d'oxychlorure de bismuth.

Selon un aspect plus particulier, l'invention a pour objet une composition pulvérulente (C₁) telle que décrite précédemment, caractérisée en ce qu'elle comprend pour 100% de sa masse une teneur massique en eau inférieure ou égale à 2% massique, de préférence inférieure ou égale à 1,5% massique, et encore plus préférentiellement inférieure ou égale à 1% massique. Selon un deuxième aspect, l'invention a pour objet une composition à usage topique (F) comprenant pour 100% de sa masse :
- De 90% à 97% massique, de préférence de 92,5% à 96,5 % massique, et encore plus préférentiellement de 94% à 96 % massique d'au moins une composition pulvérulente (C₁) telle que définie précédemment,
- De 3% à 10% massique, de préférence de 3,5% à 7,5% massique, et encore plus particulièrement de 4% à 6% massique d'au moins un agent liant gras (AL) liquide à 20°C. L'expression "à usage topique" utilisée dans la définition de la composition (F) signifie que ladite composition à usage topique est mise en oeuvre par application sur la peau du corps et du visage, sur les cheveux, sur le cuir chevelu ou sur les muqueuses, qu'il s'agisse d'une application directe dans le cas d'une formulation cosmétique, dermocosmétique, dermo-pharmaceutique ou pharmaceutique ou d'une application indirecte par exemple dans le cas d'un produit d'hygiène corporelle sous forme de lingette en textile ou en papier ou de produits sanitaires destinés à être en contact avec la peau ou les muqueuses.

Dans le cadre de la présente invention, on désigne par « agent liant gras » une substance ou une composition chimique grasse, lipophile, qui a pour fonction d'agglomérer en une masse solide des particules sous forme de poudre ou de granulats, de manière à diminuer les risques de fragmentation de celles-ci sous l'impact de chocs et à en assurer un prélèvement aisé. Selon un aspect particulier, l'invention a pour objet une composition à usage topique (F) telle que décrite précédemment, caractérisée en ce que l'agent liant gras (AL) est sélectionné parmi les éléments du groupe constitué par :
i) Les compositions comprenant au moins une huile de silicone, au moins une cire de silicone et au moins une résine de silicone, et optionnellement au moins une gomme de silicone et au moins une phényldimethicone, telle que définie dans la demande internationale de brevet publiée sous le numéro WO 93/17660 A1,
ii) Les alcanes ramifiés liquides à 20°C comme par exemple l'isododécane, ou 2,2,4,6,6-Pentamethylheptane (Numéros CAS : 31807-55-3/93685-81-5/13475-82-6), l'isohexadécane CAS = (93685-80-4) ou 2,2,4,4,6,8,8-heptaméthyl nonane (CAS = 4390-04-9),
iii) Les huiles minérales, liquides à 20°C et à pression atmosphérique, comme par exemple l'huile de paraffine, les alcanes non ramifiés comme par exemple le n-décane, le n-undécane, le n-dodécane, le n-tridécane, le n-tétradécane, le n-pentadécane, le n-hexadécane, le n-heptadécane.
iv) Les huiles d'origine végétale à base de monoglycérides et/ou de diglycérides et/ou de triglycérides, on désigne les substances chimiques comprenant des monoglycérides de formule (A₁) et/ou de formule (A'₁), et/ou des diglycérides de formule (A₂) et/ou de formule (A'₂), et/ou des triglycérides de formule (A₃) dont les matières premières nécessaires à leur préparation sont des végétaux :

   CH₃- (CH₂)ₓ₁- C(O)-O-CH₂-CH(OH)-CH₂-OH (A₁)

   HO-CH₂- CH[O-C(O)-(CH₂)ₓ₂-CH₃]-CH₂-O-H (A'₁)

   CH₃- (CH₂)ₓ₃- C(O)-O-CH₂-CH(OH)-CH₂-O-C(O)-(CH₂)ₓ₄-CH₃ (A₂)

   CH₃- (CH₂)ₓ₅- C(O)-O-CH₂-CH[O-C(O)-(CH₂)ₓ₆-CH₃]-CH₂-O-H (A'₂)

   CH₃- (CH₂)ₓ₇- C(O)-O-CH₂-CH[O-C(O)-(CH₂)ₓ₈-CH₃]-CH₂-O-C(O)-(CH₂)ₓ₉-CH₃ (A₃)

   avec x1, x2, x3, x4, x5, x6, x7, x8 et x9, identiques ou différents, représentent un nombre entier compris entre 7 et 23.
   Parmi les huiles d'origine végétale à base de monoglycérides et/ou de ditriglycérides et/ou de triglycérides telles que définies précédemment, on peut citer par exemple l'huile d'amande douce, l'huile de coprah, l'huile de ricin, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile de pastel, l'huile de bourrache, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyaux d'abricot, l'huile de calophyllum, l'huile de symbrium, l'huile d'avocat, l'huile de calendula, l'huile de chanvre, les huiles issues de fleurs, les huiles issues de légumes, les triglycérides obtenus par réaction d'estérification entre un acide gras d'origine végétale et le glycérol comme par exemple le tri-isostearate de glycérol, le caprylic capric triglycéride commercialisé sous l'appellation DUB MCT par la société STEARINERIE DUBOIS, les triglycérides obtenus par réaction d'estérification entre les acides gras comportant sept atomes de carbone et le glycérol commercialisé sous l'appellation DUB THG par la société STEARINERIE DUBOIS, les triglycérides obtenus par réaction d'estérification entre les acides gras comportant 22 atomes de carbone et le glycérol commercialisé sous l'appellation SYNCROWAX HRC par la société CRODA.
v) Les esters gras liquides à 20°C comme par exemple l'isostéaryl néopentanoate, le dicaprylyl caprate, le stéarate d'isocétyle, l'isopropyl myristate, l'hexadecyl adipate
vi) Les isoalcanols de formule (II) :

   (CH₃)(CH₃)CH-(CH₂)r-CH₂-OH (II),

   formule (II) dans laquelle r représente un nombre entier compris entre 8 et 20, par exemple les radicaux isodécyle, isoundécyle, isododécyle, isotridécyle, isotétradécyle, isopentadécyle, isohexadécyle, isopentadécyle, isooctadécyle, isononadécyle, isoeicosyle ou isodocosyle ;
vii) Les alcools de Guerbet, de formule (III) :

   CH(CsH₂s₊₁)(CₜH₂ₜ₊₁)-CH₂-OH (III)

   formule (III) dans laquelle t est un nombre entier compris entre 6 et 18, s est un nombre entier compris entre 4 et 18 et la somme s + t est supérieure ou égale à 10, et inférieure ou égale à 22, par exemple les radicaux 2-butyl octyle, 2-butyl décyle, 2-hexyl octyle, 2-hexyl décyle, 2-octyldécyle, 2-hexyl dodécyle, 2-octyl dodécyle, 2-décyl tétradécyle, 2-dodécyl hexadécyle, 2-tétradécyl octadécyle.
viii) Les alcools gras liquides à 20°C comme par exemple l'alcool oléique ou (Z)-Octadec-9-en-1-ol, l'alcool linoléique ou cis,cis-9,12-Octadecadien-1-ol, l'alcool linolénique ou (9Z, 12Z, 15Z)-9,12,15-Octadecatrien-1 -ol
ix) Les dérivés siliconés comme par exemple la diméthicone, le cyclopentasiloxane, la vinyl diméthicone, la caprylyl méthicone, la diphényl diméthicone

Selon un aspect particulier, l'agent liant gras (AL) liquide à 20°C est choisi parmi les éléments du groupe constitué par la diméthicone, la cyclopentasiloxane, la vinyl diméthicone, la caprylyl methicone, la diphenyldimethicone, l'isohexadecane, l'isododecane, l'huile de paraffine, l'huile de ricin, l'huile d'amande douce, l'huile de macadamia, le capryl-caprylyl triglycéride, le dicaprylyl caprate, l'isostéaryl néopentanoate, l'octyldodécanol, l'hexyldécanol.

Selon un aspect plus particulier, l'agent liant gras est choisi parmi les éléments du groupe constitué par l'huile de ricin, l'octyldodécanol, la diméthicone, le cétéaryl éthyl hexanoate, l'isopropyl myristate.

La composition à usage topique (F) objet de la présente invention se présente notamment sous la forme de fards à joues, de fards à paupières, de poudres pour le maquillage du visage, de poudres corporelles (parfumées et/ou désodorisantes), y compris de poudres pour les pieds et pour les mains.

La composition à usage topique (F) se présentant sous forme d'une poudre compacte et objet de la présente invention, peut comprendre des additifs habituellement mis en oeuvre dans le domaine des formulations à usage topique, en particulier cosmétiques, dermocosmétiques, pharmaceutiques ou dermo-pharmaceutiques, et choisi parmi des agents antiseptiques, des agents astringents, des filtres solaires, des agents cicatrisants, des agents anti-radicaux libres, des vitamines ; des agents adoucissants, des agents émollients, des agents hydratants (glycérol, sorbitol, etc.), des agents éclaircissant de la peau, des parfums, des agents de consistance.

Comme exemples d'agents déodorants éventuellement présents dans la composition à usage topique (F) objet de la présente invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol, le caprylate de glycérol, le caprate de polyglycérol ; le 1,2-décanediol ; le 1,3-propanediol ; l'acide salicylique ; le bicarbonate de sodium ; les cyclodextrines ; les zéolithes métalliques ; le Triclosan^{™} ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Parmi les agents de protection contre les rayonnements ultra-violets du soleil que peut comprendre la composition à usage topique (F) objet de la présente invention, on peut citer les pigments, les filtres organiques solaires et les filtres inorganiques solaires.

Comme pigments utilisés comme agent de protection contre les rayonnements ultra-violets du soleil que peut comprendre la composition à usage topique (F) objet de la présente invention, il y a par exemple le dioxyde de titane, les oxydes de fer marrons, les oxydes de fer jaunes, les oxydes de fer noirs, ou les oxydes de fer rouges ou encore les pigments nacrés blancs ou colorés tels que les Mica-Titane.

Comme filtres organiques solaires utilisés comme agent de protection contre les rayonnements ultra-violets du soleil que peut comprendre composition à usage topique (F) objet de la présente invention, il y a par exemple:
- Ceux de la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Ceux de la famille des dérivés de l'acide anthranilique comme l'homomenthyl-N-acétyl anthranilate;
- Ceux de la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle;
- Ceux de la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle;
- Ceux de la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 2-éthylhexyl 4'-phényl benzophénone-2-carboxylate, la 2-hydroxy 4-n-octyloxy benzophénone, la 4-hydroxy 3-carboxy benzophénone ; le 3-(4'-méthylbenzylidène) d,l-camphre, le 3-(benzylidène)-d,l-camphre, le benzalkonium méthosulfate camphre ; l'acide urocanique, l'urocanate d'éthyle ;
- Ceux de la famille des dérivés de l'acide sulfonique comme l'acide 2-phényl benzimidazole-5-sulfonique et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy) 1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl-5-méthyl benzoxazole, le 2,2'-hydroxy-5-méthylphényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbomylidène)-3-pentan-2-one ; le 2-(4-diethylamino 2-hydroxy benzoyl) benzoic acid hexyl ester, le 2,4-bis{[4-(2-ethylhexyloxy)-2-hydroxy] phenyl}-6-(4-methoxy phenyl) 1,3,5-triazine, le 2,4,6-tris[4-(2-ethylhexyloxycarbonyl) anilino]-1,3,5-triazine, le 2-ethylhexyl dimethoxybenzylidene dioxoimidazolidine propionate, la famille des dérivés du diphénylacrylate comme le 2-éthylhexyl-2-cyano-3,3-diphényl-2-propènoate, l'éthyl-2-cyano-3,3-diphényl-2-propènoate;
- Ceux de la famille des polysiloxanes comme le malonate de benzylidène siloxane.

Comme filtres inorganiques solaires utilisés comme agent de protection contre les rayonnements ultra-violets du soleil que peut comprendre composition à usage topique (F) objet de la présente invention, il y a par exemple: les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Parmi les agents anti-oxydants que peut comprendre la composition à usage topique (F) objet de la présente invention, on peut citer l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S (nom INCI: Tetrasodium Glutamate Diacetate).

Parmi les principes actifs que peut comprendre la composition à usage topique (F) objet de la présente invention, on peut citer :
- les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés); les composés montrant une action apaisante notamment le SEPICALM^{™} S, l'allantoïne et le bisabolol ; les agents anti-inflammatoires ; les composés montrant une action hydratante comme l'urée, les hydroxyurées, le glycérolglucoside, le diglycérolglucoside, les polyglycérylglucosides, le glycérol, le diglycérol, le xylitylpolyglucoside commercialisé sous le nom de marque AQUAXYL^{™} ; les extraits végétaux riches en polyphénols comme les extraits de raisin, les extraits de pin, les extraits de vin, les extraits d'olives; les composés montrant une action amincissante ou lipolytique comme la caféine ou ses dérivés, l'ADIPOSLIM^{™}, l'ADIPOLESS^{™}, la fucoxanthine; les protéines N-acylées; les peptides N-acylés comme le MATRIXIL^{™}; les acides aminés N-acylés; les hydrolysâts partiels de protéines N-acylés; les acides aminés; les peptides; les hydrolysâts totaux de protéines; les extraits de soja, par exemple la Raffermine^{™}; les extraits de blé par exemple la TENSINE^{™} ou la GLIADINE^{™}; les extraits végétaux, tels que les extraits végétaux riches en tanins, les extraits végétaux riches en isoflavones ou les extraits végétaux riches en terpènes; les extraits d'algues d'eau douce ou marine; les extraits de plantes marines ; les extraits marins en général comme les coraux; les cires essentielles ; les extraits bactériens ; les céramides ; les phospholipides ; les composés montrant une action antimicrobienne ou une action purifiante, comme le LIPACIDE^{™} C8G, le LIPACIDE^{™} UG, le SFPICONTROL^{™} A5; l'OCTOPIROX^{™} ou le SENSIVA^{™} SC50 ; les composés montrant une propriété énergisante ou stimulante comme le PHYSIOGENYL^{™}, le panthénol et ses dérivés comme le SFPICAP^{™} MP ; les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™} , le SURVICODE^{™} ; les actifs anti-photo vieillissement; les actifs protecteurs de l'intégrité de la jonction dermo-épidermique; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés); les actifs améliorant la microcirculation cutanée, par exemple les veinotoniques; les actifs drainants; les actifs à visée décongestionnante comme les extraits de ginko biloba, de lierre, de marron d'inde, de bambou, de ruscus, de petit houx, de centalla asiatica, de fucus, de romarin, de saule ; les agents de bronzage ou de brunissement de la peau, comme par exemple la dihydroxyacétone (DHA), l'érythrulose, l'aldéhyde mésotartrique, le glutaraldéhyde, le glycéraldéhyde, l'alloxane, la ninhydrine, les extraits végétaux comme par exemple les extraits de bois rouges du genre Pterocarpus et du genre Baphia comme le *Pteropcarpus santalinus,* le *Pterocarpus osun,* le *Pterocarpus soyauxii,* le *Pterocarpus erinaceus,* le *Pterocarpus indicus* ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, comme par exemple les caroténoïdes (et plus particulièrement le bêta-carotène et le gamma-carotène), le produit commercialisé sous le nom de marque « Carrot oil » (Nom INCI: *Daucus Carota, helianthus annuus Sunflower oil*) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, comme par exemple le produit commercialisé sous le nom de marque « SunTan Accelerator^{™} » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze^{™} (nom INCI: Acetyl Tyrosine, Monk's pepper extract (Vitex Agnus-castus) par la société Mibelle qui contient de l'acétyl tyrosine, produit commercialisé sous le nom de marque Unipertan VEG-24/242/2002 (nom INCI: butylène glycol and Acetyl Tyrosine and hydrolyzed vegetable protein and Adenosine triphosphate) par la société UNIPEX, le produit commercialisé sous le nom de marque « Try-Excell^{™} » (nom INCI: Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze^{™} » (nom INCI: hydrolyzed wheat protein and acetyl tyrosine and copper gluconate) par la société Alban Muller, le produit commercialisé sous le nom de marque Tyrostan^{™} (nom INCI: potassium caproyl tyrosine) par la société Synerga, le produit commercialisé sous le nom de marque Tyrosinol (nom INCI: Sorbitan Isostearate, glyceryl oleate, caproyl Tyrosine) par la société Synerga, le produit commercialisé sous le nom de marque InstaBronze^{™} (nom INCI: Dihydroxyacetone and acetyl tyrosine and copper gluconate) commercialisé par la société Alban Muller, le produit commercialisé sous le nom de marque Tyrosilane (nom INCI: méthylsilanol and acétyl tyrosine) par la société Exsymol; les peptides connus pour leur effet d'activation de la mélanogénèse comme par exemple le produit commercialisé sous le nom de marque Bronzing SF Peptide powder (nom INCI: Dextran and Octapeptide-5) par la société Infinitec Activos, le produit commercialisé sous le nom de marque Melitane (nom INCI: Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions^{™} (nom INCI: Butylène glycol, Palmitoyl Tripeptide-40) par la société LIPOTEC, les sucres et les dérivés de sucres comme par exemple le produit commercialisé sous le nom de marque Tanositol^{™} (nom INCI: inositol) par la société Provital, le produit commercialisé sous le nom de marque Thalitan^{™} (ou Phycosaccharide^{™} AG) par la société CODIF international (nom INCI: Aqua and hydrolyzed algin (Laminaria Digitata) and magnésium sulfate and manganèse sulfate) contenant un oligosaccharide d'origine marine (acide guluronique et acide mannuronique chélatés avec les ions magnésium et manganèse), le produit commercialisé sous le nom de marque Melactiva^{™} (nom INCI: Maltodextrin, Mucuna Pruriens Seed extract) par la société Alban Muller, les composés riches en flavonoïdes comme par exemple le produit commercialisé sous le nom de marque « Biotanning » (nom INCI: Hydrolyzed citrus Aurantium dulcis fruit extract) par la société Silab et connu pour être riche en flavonoïdes de citron (de type hespéridines);

Parmi les agents de texture que peut comprendre la composition à usage topique (F) objet de la présente invention, on peut citer la lauroyl lysine commercialisée sous l'appellation AMINOHOPE^{™}LL par la société AJINOMOTO, l'octenyl starch succinate commercialise sous l'appellation DRYFLO^{™} par la société NATIONAL STARCH, le myristyl polyglucoside commercialisé par SEPPIC sous l'appellation MONTANOV^{™} 14, les fibres de cellulose, les fibres de coton, les fibres de chitosane.

Parmi les ***substances parfumantes ou aromatisantes*** que peut comprendre la composition à usage topique (F) objet de la présente invention, on peut citer *:*
- les extraits de différentes parties des végétaux: la fleur, la feuille, la tige ou le bois, l'écorce ou la mousse de bois, le fruit ou la graine, la racine,
- ***les extraits* de fleurs** comme la rose, le jasmin, la tubéreuse, la fleur de champaca, la fleur de frangipanier, la fleur d'Ylang-ylang, la fleur de lotus, la fleur de mimosa, la fleur d'oeillet, la fleur d'osmanthus, la fleur d'acacia, la fleur d'oranger doux, la fleur d'oranger amer ou néroli, la fleur de narcisse, la lavande, le gardénia,
- les extraits de **feuilles, de mousse, d'écorce, de résine ou de bourgeons** comme les bourgeons de cassis, la mousse de chêne, la mousse de hêtre le lichen, les feuilles d'acacia, les feuilles de basilic, les feuilles de valériane, les feuilles de gentiane, les feuilles de violette, les feuilles de géranium, les feuilles de labdanum, le romarin, les feuilles de patchouli, les feuilles de verveine, l'écorce de cannelle, l'écorce de frêne, l'écorce de cassia, l'écorce de cascarille, le bois de santal, le bois de cèdre, le bois de palissandre, le bois d'aloès, l'écorce de bouleau, bois de gaïac, le baume du Pérou, la baume du tolu, la résine de Benjoin, la myrrhe, la résine de labdanum, la résine d'élémi, l'oliban, l'opoponax, le guggul, d'aiguilles et de branches de pin ou d'épicéa ou de sapin,
- les extraits de **d'herbes ou de graminées** comme l'estragon, le lemon grass, la sauge, le thym,
- les extraits de fruits ou de graines comme la fève de tonka, les gousses de vanille, la cardamone, la coriandre, la badiane, l'amande amère, le cumin, les clous de girofle, les baies de genièvre, les agrumes tels le citron, l'orange dont la linette et la bergamote, la mandarine,
- les extraits de **racines** comme les racines d'angélique, de céleri, de cardamone, d'iris, d'acore, de cactus, de vétiver,
- les extraits aromatiques, les absolues, les alcoolats et **les huiles essentielles.**

On en entend par huile essentielle un produit odorant, généralement de composition complexe, conforme à la réglementation cosmétique, obtenue à partir d'une matière première végétale botaniquement définie. Le mode d'obtention de l'huile essentielle est décrit dans la norme ISO 9235 et ces huiles essentielles peuvent éventuellement avoir subi un traitement ultérieur approprié comme avoir été déterpénées, désesquiterpénées, ou rectifiées.

Comme huile essentielle, que l'on peut associer à la composition à usage topique (F) objet de la présente invention, on peut citer par exemple l'huile essentielle d'achillée millefeuille, l'huile essentielle d'acore calamus, l'huile essentielle d'ail, l'huile essentielle d'ajowan, l'huile essentielle d'amyris, l'huile essentielle d'aneth, l'huile essentielle d'anis, l'huile essentielle d'angélique, l'huile essentielle d'arbre à thé, l'huile essentielle de basilic, l'huile essentielle de Bay Saint Thomas, l'huile essentielle de benjoin, l'huile essentielle de bergamote, l'huile essentielle de bois de gaïac, l'huile essentielle de bois de Hô, l'huile essentielle de bois de rose, l'huile essentielle de bois de santal, l'huile essentielle de bois de siam, l'huile essentielle de bouleau noir, l'huile essentielle de camomille, l'huile essentielle de camphrier, l'huile essentielle de cannelle, l'huile essentielle de cardamone, l'huile essentielle de carotte, l'huile essentielle de carvi, l'huile essentielle de cèdre, l'huile essentielle de céleri, l'huile essentielle de criste marine, l'huile essentielle de ciste, l'huile essentielle de citron, l'huile essentielle de citronnelle, l'huile essentielle de clémentine, l'huile essentielle de combawa, l'huile essentielle de copahu, l'huile essentielle de coriandre, l'huile essentielle de cryptomeria, l'huile essentielle de cumin, l'huile essentielle de curcuma, l'huile essentielle de cyprès, l'huile essentielle d'encens, l'huile essentielle d'épinette, l'huile essentielle d'estragon, l'huile essentielle de fenouil, l'huile essentielle de fragonia, l'huile essentielle de galbanum, l'huile essentielle de gaulthérie (wintergreen), l'huile essentielle de genévrier, l'huile essentielle de géranium, l'huile essentielle de gingembre, l'huile essentielle de clou ou de feuille de girofle, l'huile essentielle d'helichryse, l'huile essentielle d'hysope, l'huile essentielle d'iary, l'huile essentielle d'inule, l'huile essentielle de katrafay, l'huile essentielle de Khella, l'huile essentielle de kunzea, l'huile essentielle de lavande, l'huile essentielle de lavandin, l'huile essentielle de mandarine, l'huile essentielle de niaouli, l'huile essentielle de menthe poivrée, l'huile essentielle d'orange, l'huile essentielle de pamplemousse, l'huile essentielle de romarin, l'huile essentielle de thym, l'huile essentielle d'Ylang Ylang, l'huile essentielle de ravintsara, l'huile essentielle de sauge, l'huile essentielle de Cabreuva, l'huile essentielle de Lemongrass, l'huile essentielle de Palmarosa, l'huile essentielle de millepertuis, l'huile essentielle de jasmin, l'huile essentielle de camomille, l'huile essentielle de mélisse, l'huile essentielle de pin, l'huile essentielle de gingembre, l'huile essentielle de persil, l'huile essentielle d'armoise, l'huile essentielle de chanvre, l'huile essentielle de houblon, ou encore l'huile essentielle de serpolet.

Parmi les « substances parfumantes ou aromatisantes» animales que l'on peut associer à la composition à usage topique (F) objet de la présente invention, on peut citer par exemple le musc, le castoreum, la civette, l'ambre gris, l'absolue de cire d'abeille, l'hyraceum. Ces substances peuvent également être reconstituées par synthèse.

Parmi les « substances parfumantes ou aromatisantes» synthétiques que l'on peut associer à la composition à usage topique (F) objet de la présente invention, on peut citer par exemple :
a) des hydrocarbures terpéniques (mono et sesquiterpènes) tels que les myrcènes (α-myrcène ou β-myrcène, et l'α-myrcène), le limonène, l'α-pinène, le β-pinène, le camphène, le cadinène, le cedrène, le famesène, le caryophyllène, le chamazulène, 1,1-diméthoxy-2,2,5-triméthyl-4-hexène, le curcumène, le crythmène, les himachelènes, le limonène, le paracymène, l'oxyde de rose, l'oxyde de tetranorlabdane commercialisé sous le nom d'Ambrox par la société Firmenich, les terpinènes et terpinolènes, les vetivènes,
b) **des esters** tels que l'acétate de benzyle, l'acétate de bornyle, l'acétate de citronellyle, l'acétate de cédryle, l'acétate de dihydromyrcényle, l'acétate de diméthyl-benzylcarbinyle, l'acétate d'ethyle, l'acétate de famésyle, l'acétate de fenchyle, l'acétate d'hexyle, l'acétate de geranyle, l'acétate d'isobutyle, l'acétate d'isononyle, l'acétate d'isopentyle, l'acétate d'isobornyle, l'acétate d'isopulégyle, l'acétate de linalyle, l'acétate de menthyle, l'acétate de méthyl phényl carbinyle, l'acétate de néryle, l'acétate de nonyle, l'acétate d'o-t-butylcyclohexyle, l'acétate de phényléthyle, l'acétate de p-tert-butylcyclohexyle, l'acétate de phényléthyle, l'acétate de prényle, l'acétate de styrallyle, l'acétate de terpenyle, l'acétate de 4-tert-butylcyclohexyle, l'acetate de vetyvéryle, l'anthralinate de methyle, le benzoate de benzyle, le benzoate d'isobutyle, le benzoate de linalyle, la coumarine, le butanoate d'ethyle, le butanoate de benzyle, le butanoate d'isoamyle, le butyrate de benzyle, le butyrate d'ethyle, le butyrate d'ethyle, le butyrate d'isoamyle, le butyrate de lynalyle, le cinnamate de butyle, le caproate d'allyle, le cinnamate d'ethyle, le formiate de benzyle, le formiate de citronellyle, l'hédione (dihydrojasmonate de méthyle), le formiate de geranyle, le formiate de methyle, le glycinate d'éthylméthylphényle, le glycolate d'allyl-amyle, l'heptanoate d'allyle, l'isobutyrate de phénoxyéthyle, l'isobutyrate de cis-3-hexényle, le méthacrylate d'isoamyle, le naphtolate d'ethyle, le néopentanoate d'hexyle, le propionate d'amyle, le propionate d'alkylcyclohexyle, le propionate d'allylcyclohexane, le propionate de lynalyle, le propionate de styralyle, le propionate de citronellyle, le salicylate de methyle, le salicylate de benzyle, le salicylate d'ethyle, le tiglate d'hexyle,
c) **des alcools et des phénols** tels que l'alcool benzoïque, l'alpha terpinéol, l'anéthol, le carotol, le chavicol, l'estragol , le cinéol, l'alcool cinnamique, le citronellol, le p-crésol, l'alcool cumique, le 3,7-diméthyl-1-octanol, le diméthyle benzyle carbinol, l'alcool fenchylique, le l'eucalyptol, le farnesol, l'eugénol, l'alcool isononylique, l'isoeugénol, le gaïacol, le géraniol, le globutol, le linanol, le menthol, le dihydromyrcénol, le nérolidol, le nérol, l'alcool phenyl éthylique, le safrol, l'isosafrol, le phytol, l'iso phytol, le terpinéol, le tétrahydrolinalol, le tétrahydromyrcénol, le thymol, le vétivérol, l'undécavertol,
d) **des aldéhydes** tels que l'aldéhyde phényl acétique, l'aldéhyde salicylique , l'aldéhyde anisique, l'aldéhyde caprylique, l'aldéhyde cinnamique, l'aldéhyde hexyl cinnamique, le bourgeonal, le citral (néral), le citronellal, l'hydroxy-citronellal, le citronellyloxyacétaldéhyde, le cyclamènaldéhyde, le cuminaldéhyde, le cyclal, le 2,4-diméthyl-3-cyclohexène-1-carboxaldéhyde (ligustral), le dodécanal, le décylaldéhyde, l'éthanal, l'octanal, le décanal, les géranials, l'hélional, le lauraldéhyde, les lactones telles que les gamma-undécalactones, le lilial, le méthyl n-nonyl acétaldéhyde, le méthyl octyl acétaldéhyde, l'undécanal, l'aldéhyde salicylique, la vanilline
e) **des cétones** tels que la benzyl acétone, la calone (7-méthyl-2H-benzo-1,5-dioxepin-3(4H)-one), la carvone, le camphre, la civéttone, les damascones, les damascénones, l'éthyl-amyl-cétone, l'éthyl-hexyl-cétone, la géranyl cetone, la jasmone, les irones, le maltol (3-hydroxy-2-methyl-4H-pyran-4-one), l'ethyl maltol, la menthone, l'iso menthone, la muscone, la méthyl hepténone, les ionones comme la methyl-ionone, la 4-méthylacétophénone, la méthylpentylcétone, la méthyl-heptylcétone, la méthyl-hexylcétone, l'alpha-isométhylionone, et la méthylcédrylcétone commercialisé sous l'appelation vertofix par la société IFF,
f) **des éthers** tels que l'anéthol, le benzyléthléther, le cédryl méthyl ether, le p-crésyl méthyl éther,
g) **des muscs artificiels** issus de différents composés nitrés, les muscs ambrette, les muscs cétones, les muscs xylène, les muscs macrocycliques,
h) **des nitriles** tels que les trimethyl- 3 , 5, 7-octane (ène) nitriles et leurs dérivés α-substitués, le citronellyl nitrile, le citronitrile, le géranyl-nitrile.

Selon un autre aspect, l'invention a pour objet l'utilisation d'un polymère de type polyélectrolyte anionique réticulé (P) tel que défini précédemment pour améliorer la résistance aux chocs d'une formulation cosmétique à usage topique (F) telle que décrite ci-dessus.

Selon un autre aspect, l'invention a pour objet un procédé de maquillage de la peau humaine comprenant au moins une étape d'application sur ladite peau humaine ou animale d'une composition à usage topique (F) telle que définie précédemment.

Selon un autre aspect, l'invention a pour objet une composition à usage topique (F) selon l'invention comprenant une quantité efficace d'au moins un filtre organique solaire et/ou d'au moins un filtre inorganique solaire, pour protéger la peau humaine contre les effets inesthétiques sur la dite peau humaine des rayons ultra-violets du soleil.

Par « effets inesthétiques sur ladite peau humaine des rayons ultra-violets du soleil », on désigne une coloration excessive non souhaitée comme les rougeurs de la peau, une évolution de la plasticité de la peau pouvant se traduire par une flétrissure de la peau, par l'apparition de rides et/ou de ridules creusées, par une desquamation superficielle communément appelée le « pelage de la peau » correspondant à la séparation de peaux mortes plus nombreuses en raison de l'exposition prolongée de la peau aux rayons ultra-violet du soleil.

Ladite étape d'application de la composition à usage topique (F) sur la peau peut être réalisée à l'aide des doigts, ou d'un applicateur comme par exemple un pinceau ou une éponge.

Les exemples suivants illustrent l'invention.

### Exemple 1 :

### 1.1. Préparation d'un terpolymère de 2-méthyl 2-[(1-oxo 2-propènyl) amino] 1-propanesulfonate d'ammonium, N,N-diméthyl acrylamide et méthacrylate de lauryle tétraéthoxylé [AMPS/DMAM/MAL(4OE) 77,4/19,2/3,4 molaire], réticulé au triméthylol propanetriacrylate (TMPTA) (polymère selon la revendication 1).

On charge dans un réacteur maintenu à 25°C sous agitation, 592g d'une solution aqueuse à 15% massique de 2-méthyl 2-[(1-oxo 2-propènyl) amino] 1-propanesulfonate d'ammonium dans un mélange tert-butanol/eau (97,5/2,5 en volume), 10,1g de N,N-diméthyl acrylamide, 4,2g de méthacrylate de lauryle tétraéthoxylé et 0,75g de triméthylol propanetriacrylate.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : Polyélectrolyte 1

### 1.2 Polyélectrolyte réticulé de 2-méthyl 2-[(1-oxo 2-propènyl) amino] 1-propanesulfonate d'ammonium, d'acrylate de (2-hydroxy éthyle), de méthacylate de stéaryle et de méthacrylate de lauryle, réticulé au triacrylate de triméthylolpropane (ATBS/HEA/MAS/MALAU : 88,1/9,9/1,0/1,0 ; Polyélectrolyte 2) (polymère selon la revendication 1).

On charge dans un réacteur maintenu à 25°C sous agitation contenant 245 g de tertio butanol :
- 33,8g d'acide 2-acrylamido 2- méthyl propanesulfonique (ATBS) ;
- 12,9g d'hydrogénocarbonate d'ammonium ;
- 2,12g d'acrylate de (2-hydroxy éthyle) (HEA) ;
- 0,62g de méthacrylate de stéaryle (MAS) ;
- 0,48g de méthacrylate de lauryle (MALAU) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures avant de refroidir. La poudre qui s'est formée en cours de polymérisation est filtrée et séchée pour obtenir le polyélectrolyte 2.

### 1.3. Préparation d'un terpolymère de 2-méthyl 2-[(1-oxo 2-propènyl) amino] 1-propanesulfonate d'ammonium, 2-hydroxyéthylacrylate et méthacrylate de lauryle tétraéthoxylé [AMPS/HEA/MAL(4OE) 77,4/19,2/3,4 molaire], réticulé au triméthylol propanetriacrylate (TMTPA) (polymère selon la revendication 1).

En mettant en oeuvre les conditions opératoires du procédé décrit dans l'exemple 1.1 précédent, on charge dans un réacteur maintenu à 25°C sous agitation, la quantité massique nécessaire d'une solution aqueuse à 15% massique de 2-méthyl 2-[(1-oxo 2-propènyl) amino] 1-propanesulfonate d'ammonium dans un mélange tert-butanol/eau (97,5/2,5 en volume) de façon à introduire 77,4 équivalents molaires de 2-méthyl 2-[(1-oxo 2-propènyl) amino] 1-propanesulfonate d'ammonium, la quantité massique nécessaire de 2-hydroxyéthylacrylate de façon à introduire 19,2 équivalents molaires de 2-hydroxyéthylacrylate, la quantité massique nécessaire de méthacrylate de lauryle tétraéthoxylé de façon à introduire 3,4 équivalents molaires de méthacrylate de lauryle tétraéthoxylé, et la quantité massique nécessaire de triméthylol propanetriacrylate de façon à obtenir la même proportion molaire de triméthylol propanetriacrylate que dans l'exemple 1.1.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : Polyélectrolyte 3.

### 1.4. Préparation d'un copolymère de 2-méthyl 2-[(1-oxo 2-propènyl) amino] 1-propanesulfonate d'ammonium, 2-hydroxyéthylacrylate [AMPS/HEA 90/10 molaire], réticulé au méthylène-bis-acrylamide (MBA) (pas un polymère selon la revendication 1).

On charge dans un bêcher, sous agitation :
- 608,8 grammes d'une solution commerciale à 50% du sel de sodium de l'acide 2-méthyl 2-[(1-oxo 2-propènyl) amino] 1-propanesulfonique,
- 72,6 grammes d'acrylate de (2-hydroxyéthyle),
- 0,18 grammes de diéthylène triamine pentacétate de sodium, et
- 0, 121 grammes de méthylène bis (acrylamide),
- le pH de la phase aqueuse précédemment décrit est ajusté à 3,5, par ajout de 0,7g d'acide 2-methyl-2 [(1-oxo-2 propènyl) amino] 1-propanesulfonique.

Parallèlement, on prépare une phase organique en introduisant dans un bêcher agité successivement :
- 220g d'Isopar M,
- 25 g de Montane 70 VG (oléate de sorbitan éthoxylé à 20 moles d'oxyde d'éthylène, commercialisé par SEPPIC) et
- 0, 2 g d'azo bis (isobutyronitrile).

La phase aqueuse est introduite progressivement dans la phase organique puis soumise à une agitation mécanique violente de type ultra-turrax commercialisé par IKA.

L'émulsion obtenue est alors transférée dans un réacteur de polymérisation. L'émulsion est soumise à un barbotage d'azote important de manière à éliminer l'oxygène et refroidit à environ 5-6 °C.

On introduit alors 10 grammes d'une solution à 1,1% en poids de matière active d'hydroperoxyde de cumène dans l'isohexadécane. Après un temps suffisant pour une bonne homogénéisation de la solution, on introduit en environ 25 minutes, 25 grammes d'une solution aqueuse de métabisulfite de sodium (solution à 0,2%). Pendant cette introduction, on laisse monter la température dans le réacteur de polymérisation jusqu'à la température finale de polymérisation puis on maintient le milieu réactionnel pendant environ 90 minutes à cette température.

L'ensemble est ensuite refroidi jusqu'à une température d'environ 35 °C pour obtenir une émulsion qui est ensuite atomisée au moyen d'un appareil de type Niro^{™} et l'on obtient le Polyélectrolyte 4 sous forme poudre.

Dans l'exemple 1.4 décrit ci-dessus, le monomère de formule (1) n'a pas été impliqué dans la réaction de polymérisation, de façon à disposer d'un polyélectrolyte anionique réticulé (Polyélectrolyte 4) destiné à comparer l'effet associé à la présence du monomère de formule (1) dans le squelette polymérique du polyélectrolyte anionique réticulé.

### Exemple 2 :

### A- Préparation de compositions compactes selon l'invention, comprenant les Polyélectrolytes 1 à 4, et de formulations comparatives.

On introduit les pigments colorés, l'agent de charge (talc) et le polyélectrolyte anionique réticulé à tester dans un broyeur à couteaux de marque Vorwerk, de modèle Thermomix TM3300.Le mélange des poudres est alors soumis à des opérations de broyage successives, 3 fois 30 secondes, à une température de 25°C, à une vitesse d'agitation de 7 700 tours/minute.

Les parois du bol sont raclées entre chaque broyage afin de bien entrainer toute la poudre lors de l'étape du broyage.

L'agent liant est ensuite réparti sur le mélange de poudres ainsi obtenu dans le bol du broyeur, et ce nouveau mélange est alors soumis à une opération de broyage 3 fois 30 secondes en raclant efficacement les parois du bol entre chaque broyage.

Une quantité optimale de 11g de ce mélange est introduit dans un godet métallique de 3 centimètres de diamètre, et un tissu de type « Bergamot » (100% polyamide) est déposé à la surface du godet.

Ce godet est ensuite placé dans une « compacteuse » manuelle, à savoir un appareil permettant de tasser les poudres présentes dans un godet en appliquant une pression déterminée.

Une pression de 130 bars est ensuite appliquée sur le godet pendant une durée de 1 seconde à une température de 25°C .

On obtient les formulations (F1), (F2), (F3), (F4), selon l'invention, ainsi que les formulations comparatives (F5), (F6), (F'1) et (F'2), telles que décrite dans les tableaux 1 et 2 ci-dessous.

**[Tableau 1]**

| | (F1) | (F2) | (F3) | (F'1) | (F'2) |
|---|---|---|---|---|---|
| Polymère (en % massique) | | | | | |
| Polyélectrolyte 1 | 0,5% | 0% | 0% | 0% | 0% |
| Polyélectrolyte 2 | 0% | 0,5% | 0% | 0% | 0% |
| Polyélectrolyte 3 | 0% | 0% | 0,5% | 0% | 0% |
| Polyélectrolyte 4 | 0% | 0% | 0% | 0,5% | 0% |
| Agent de charge (en % massique) : talc | 88,9% | 88,9% | 88,9% | 88,9% | 89,4% |
| Pigment : | 6,1% | 6,1% | 6,1% | 6,1% | 6,1% |
| Sunpuro TiO2 C47-5001 (INCI = CI 77891) (en % massique) | | | | | |
| Agent liant gras (en % massique) : | 4,5% | 4,5% | 4,5% | 4,5% | 4,5% |
| Eutanol^{™}G 16 (INCI= hexyldecanol) | | | | | |

**[Tableau 2]**

| | (F4) | (F1) | (F5) | (F6) |
|---|---|---|---|---|
| Polymère (en % massique) | | | | |
| Polyélectrolyte 1 | 0,2% | 0,5% | 1% | 2% |
| Agent de charge (en %massique) : talc | 89,2% | 88,9% | 88,4% | 87,4% |
| Pigment coloré (en % massique) : | 6,1% | 6,1% | 6,1% | 6,1% |
| Sunpuro TiO2 C47-5001 (INCI = CI 77891 | | | | |
| Agent liant gras (en % massique) : | 4,5% | 4,5% | 4,5% | 4,5% |
| Eutanol^{™}G 16 (INCI= hexyldecanol) | | | | |

### B- Mise en évidence des propriétés et des caractéristiques des formulations compactes selon l'invention comparativement à celles de l'état de la technique.

Les formulations (F1) à (F6), et les formulations (F'1) et (F'2) comparatives précédemment préparées sont ensuite évaluées comme suit :
- Mesure de leur résistance aux chocs selon une méthode d'un « test de chute »,
- évaluation du transfert sur la peau à température ambiante (25°C)
- évaluation du transfert sur la peau après stockage en atmosphère humide à 50°C

### Protocole expérimental pour l'évaluation de la résistance aux chocs selon une méthode d'un « test de chute » pour les formulations (F1) à (F6), et les formulations (F'1) et (F'2)

- *Principe :* déterminer le nombre de chutes successives à partir duquel apparaissent des brisures et/ou un effritement de la surface de la formulation compacte à tester.
- *Matériel* : godet métallique de 3 centimètres de diamètre contenant les formulations compactes à tester, guide tubulaire pour la chute
- *Mode opératoire* : les formulations à tester sont préparées selon le mode opératoire décrit ci-dessus, et sont obtenues dans des godets de 3 centimètres de diamètre. Chaque godet est mis à une hauteur de 20 centimètres du sol et est lâché x fois le long d'un guide tubulaire depuis ladite hauteur. L'expérimentateur observe après chaque chute, la surface de la formulation présente dans le godet et note selon le cas « absence de brisures ou d'effritement à la surface » ou « présence de brisures ou d'effritement à la surface ». L'expérimentateur détermine alors le nombre de chutes consécutives nécessaire pour observer pour la première fois la « présence de brisures ou d'effritement à la surface ». Le test est répété 3 fois pour chaque formulation.

### Protocole expérimental pour l'évaluation de la qualité du transfert sur la peau pour les formulations (F1) à (F6), et la formulation (F'1) à 25°C et à 50°C

- *Principe :* qualifier le transfert de la formulation compacte à tester sur le doigt de l'expérimentateur à 25°C et à 50°C, en estimant la suffisance de la quantité recueillie après un passage du doigt sur le godet.
- *Matériel* : godet métallique de 3 centimètres de diamètre contenant les formulations compactes à tester
- *Mode opératoire* : les formulations à tester sont préparées selon le mode opératoire décrit ci-dessus, et sont obtenues dans des godets de 3 centimètres de diamètre. Avant chaque test, l'expérimentateur se lave les mains avec un savon et se sèche les mains pour enlever l'humidité provenant du rinçage. L'expérimentateur passe ensuite le doigt à la surface d'un godet comprenant la formulation à tester pour prélever celle-ci. L'expérimentateur détermine alors la suffisance de la quantité recueillie avec les critères suivants : « préhension insuffisante », « préhension moyenne » et « bonne préhension ».
- *Conditions de mesure:* les formules sont testées après stockage pendant 7 jours à 25°C et à humidité ambiante et à 50°C humide : pour ces mesures les godets sont placés au-dessus d'un lit d'eau, dans une enceinte fermée placée dans une étuve à 50°C pendant 7 jours. Les mesures sont réalisées à 1 jour, 3 jours, et 7 jours

Les résultats de ces évaluations sont consignés dans les tableaux 3 et 4 suivants.

**[Tableau 3]**

| | (F1) | (F2) | (F3) | (F'1) | (F'2) |
|---|---|---|---|---|---|
| *Test de chute* : | | | | | |
| Nombre de chutes consécutives avant apparition de brisures | 8 | 7 | 5 | 1 | 5 |
| Qualité de la préhension sur la peau à 25°C | | | | | |
| Appréciation | bonne préhension | bonne préhension | bonne préhension | bonne préhension | bonne préhension |
| Qualité de la préhension sur la peau à 50°C | | | | | |
| Appréciation | bonne préhension | bonne préhension | bonne préhension | bonne préhension | bonne préhension |

**[Tableau 4]**

| | (F4) | (F1) | (F5) | (F6) |
|---|---|---|---|---|
| *Test de chute* : | | | | |
| Nombre de chutes consécutives avant apparition de brisures | 3 | 8 | 7 | 7 |
| Qualité de la préhension sur la peau à 25°C | | | | |
| Appréciation | bonne préhension | bonne préhension | bonne préhension | bonne préhension |
| Qualité de la préhension sur la peau à 50°C | | | | |
| Appréciation | bonne préhension | bonne préhension | mauvaise préhension | mauvaise préhension |

### Commentaires et conclusions

Les résultats sont jugés satisfaisants lorsque :
- Le nombre de chutes consécutives après lesquelles l'expérimentateur n'observe pas la « présence de brisures ou d'effritement à la surface », est supérieur ou égal à 5, et
- La qualité de la préhension à 25°C est jugée comme « bonne préhension »
- La qualité de la préhension à 50°C est jugée comme « bonne préhension »

Les formulations (F1), (F2) et (F3) comprenant 0,5% massique respectivement de Polyélectrolyte 1, de Polyélectrolyte 2 et de Polyélectrolyte 3 montrent une résistance aux chocs souhaitée (nombre de chutes avant l'observation de brisures respectivement de 8, 7 et 5) et une qualité de préhension jugée comme une « bonne préhension » à 25°C et 50°C. Par contre, la formulation (F' 1) comprenant un copolymère à base du sel de sodium de l'acide 2-méthyl 2-[(1-oxo 2-propènyl) amino] 1-propanesulfonique et de 2-hydroxyéthyl acrylate, réticulé au méthylène-bis-acrylamide, mais ne comprenant pas de monomère de formule (I) telle que définie ci-dessus, présente une mauvaise résistance aux chocs puisqu'il suffit d'une seule chute pour voir apparaître des brisures à la surface de la formulation compacte (F' 1).

Les formulations comparatives (F5) et (F6), qui comprennent une proportion massique en polyélectrolyte anionique réticulé (P) supérieure à 0,7% dans la composition (C₁), se caractérisent par une mauvaise préhension sur la peau selon le test à 50°C.

La formulation comparative (F'₂), qui se caractérise par l'absence de polymère de type polyélectrolyte anionique réticulé (P), montre une résistance non améliorée aux chocs.

## Revendications

1. Composition pulvérulente (C₁) sous forme de poudre comprenant pour 100% de sa masse :
i) de 84,3% à 95,8% massique d'au moins un agent de charge (AC)
ii) de 4% à 15% massique d'au moins un pigment coloré (PC)
- iii) de 0,2% à 0,7% massique d'au moins un polymère de type polyélectrolyte anionique réticulé (P) qui comporte pour 100% molaire, de 65% molaire à 95% molaire d'unités monomériques issues de l'acide 2-méthyl 2-[(1-oxo 2-propènyl) amino] 1-propanesulfonique partiellement ou totalement salifié, de 4,8% molaire à 25% molaire d'unités monomériques issues d'au moins un monomère neutre choisi parmi les éléments du groupe constitué par le 2-hydroxy éthyl acrylate, le 2-hydroxy éthyl méthacrylate, les N,N-dialkyl acrylamides dans lesquels chacun des groupes alkyle comportent entre un et quatre atomes de carbones, et de 0,1% à 5% molaire d'unités monomériques issues d'au moins un monomère de formule (I) : 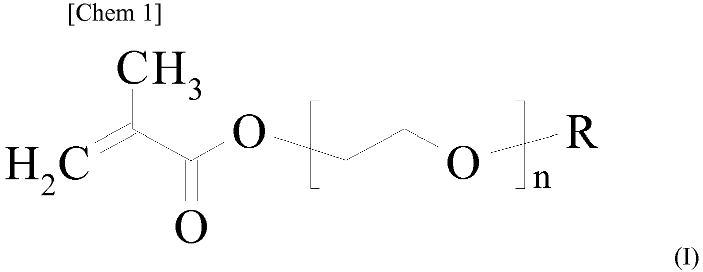 dans laquelle R représente un radical alkyle linéaire ou ramifié comportant de huit à vingt atomes de carbone et n représente un nombre supérieur ou égal à zéro et inférieur ou égal à vingt, et de 0,1% à 5% molaire d'unités monomériques issues d'au moins un monomère de réticulation diéthylénique ou polyéthylénique

2. Composition pulvérulente (C₁) selon la revendication 1 , **caractérisée en ce que** dans le polymère de type polyélectrolyte anionique réticulé (P), ledit monomère neutre est choisi parmi les éléments du groupe constitué par le 2-hydroxy éthyl acrylate ou le N,N diméthyl acrylamide.

3. Composition pulvérulente (C₁) selon l'une des revendications 1 ou 2, **caractérisée en ce que** dans le polymère de type polyélectrolyte anionique réticulé (P), ledit monomère de formule (I) est choisi parmi les éléments du groupe constitué par le méthacrylate de lauryle tétraéthoxylé de formule (I₁) correspondant à la formule (I) dans laquelle R représente le radical n-dodécyle et dans laquelle n est égal à quatre, par le méthacrylate de lauryle de formule (I₂), correspondant à la formule (I) dans laquelle R représente un radical n-dodécyle et dans laquelle n est égal à zéro, par le méthacrylate d'isodécyle de formule (I'₂), correspondant à la formule (I) dans laquelle R représente un radical isododécyle et dans laquelle n est égal à zéro, et par le méthacrylate de stéaryle de formule (I₃), correspondant à la formule (I) dans laquelle R représente un radical n-octadécyle et dans laquelle n est égal à zéro.

4. Composition pulvérulente (C₁) selon l'une des revendications 1 à 3, **caractérisée en ce que** dans le polymère de type polyélectrolyte anionique réticulé (P), le monomère de réticulation diéthylénique ou polyéthylénique est choisi parmi le diméthacrylate d'éthylèneglycol, le tétraallyloxyéthane, le diacrylate d'éthylèneglycol, le diallyl urée, le triallylamine, le triméthylol propanetriacrylate, le méthylène-bis(acrylamide) ou un mélange de ces composés.

5. Composition pulvérulente (C₁) selon l'une des revendications 1 à 4, **caractérisée en ce que** ledit polymère de type polyélectrolyte anionique réticulé (P) est un polymère de l'acide 2-méthyl 2-[(1-oxo 2-propènyl) amino] 1-propanesulfonique partiellement ou totalement salifié sous forme de sel d'ammonium, du N,N diméthyl acrylamide et du méthacrylate de lauryle tétraéthoxylé de formule (I₁), réticulé au triméthylolpropanetriacrylate.

6. Composition pulvérulente (C₁) selon la revendication 5, **caractérisée en ce que** ledit polymère de type polyélectrolyte anionique réticulé (P) comporte pour 100% molaire :
- de 65% molaire à 95% molaire d'unités monomériques issues de l'acide 2-méthyl 2-[(1-oxo 2-propènyl) amino] 1-propanesulfonique salifié sous forme d'ammonium;
- de 4,8% molaire à 25% molaire d'unités monomériques issues du N,N-diméthyl acrylamide,
- de 0,1% à 5% molaire d'unités monomériques issues du méthacrylate de lauryle tétraéthoxylé de formule (I₁),
- de 0,1% à 5% molaire d'unités monomériques issues du triméthylolpropanetriacrylate.

7. Composition pulvérulente (C₁) telle que définie à l'une ou quelconque des revendications 1 à 4, **caractérisée en ce que** ledit polymère de type polyélectrolyte anionique réticulé (P) est un polymère de l'acide 2-méthyl 2-[(1-oxo 2-propènyl) amino] 1-propanesulfonique partiellement ou totalement salifié sous forme de sel de sodium, du 2-hydroxy éthyl acrylate, du méthacrylate de lauryle de formule (I₂) ou du méthacrylate d'isodécyle de formule (I'₂) et du méthacrylate de stéaryle de formule (I₃) dans un ratio molaire (I₂)/(I₃) ou (I'₂)/(I₃) supérieur ou égal à 1/10 et inférieur ou égal à 10/1, et réticulé au triméthylolpropanetriacrylate.

8. Composition pulvérulente (C₁) telle que définie à la revendication 7, **caractérisée en ce que** ledit polymère de type polyélectrolyte anionique réticulé (P) comporte pour 100% molaire :
- de 70% molaire à 95% molaire d'unités monomériques issues de l'acide 2-méthyl 2-[(1-oxo 2-propènyl) amino] 1-propanesulfonique salifié sous forme de sodium ;
- de 4,8% molaire à 20% molaire d'unités monomériques issues du 2-hydroxy éthyl acrylate,
- de 0,1% molaire à 5% molaire d'unités monomériques issues du méthacrylate de lauryle de formule (I₂) ou du méthacrylate d'isodécyle de formule (I'₂), et du méthacrylate de stéaryle de formule (I₃), dans un ratio molaire (I₂)/(I₃) ou (I'₂)/(I₃) supérieur ou égal à 1/6 et inférieur ou égal à 6/1,
- de 0,1% molaire à 5% molaire de triméthylolpropanetriacrylate.

9. Composition pulvérulente (C₁) telle que définie à l'une ou quelconque des revendications 1 à 8, **caractérisée en ce que** l'agent de charge (AC) est choisi parmi les éléments du groupe constitué par les charges de type lamellaire minéral, les charges de type lamellaire organique, les charges de type sphérique minéral et les charges de type sphérique organique.

10. Composition pulvérulente (C₁) telle que définie à l'une ou quelconque des revendications 1 à 9, **caractérisée en ce que** le pigment coloré (PC) est choisi parmi les éléments du groupe constitué par, les pigments minéraux, les pigments organiques et les pigments nacrés.

11. Composition pulvérulente (C₁) telle que définie à l'une ou quelconque des revendications 1 à 10, **caractérisée en ce qu'**elle comprend pour 100% de sa masse une teneur massique en eau inférieure ou égale à 2% massique.

12. Composition à usage topique (F) comprenant pour 100% de sa masse :
i) De 90% à 97% massique d'au moins une composition pulvérulente (C₁) telle que définie à l'une ou quelconque des revendications 1 à 11
ii) De 3% à 10% massique d'au moins un agent liant gras (AL) liquide à 20°C.

13. Composition à usage topique (F) telle que définie à la revendication 12 **caractérisée en ce que** l'agent liant (AL) est sélectionné parmi les éléments du groupe constitué par l'huile de ricin, l'Octyldodecanol, la Dimethicone, le Cetearyl Ethylhexanoate, l'isopropyl Myristate.

14. Utilisation d'un polymère de type polyélectrolyte anionique réticulé (P) tel que défini à l'une ou quelconque des revendications 1 à 8 pour améliorer la résistance aux chocs d'une formulation cosmétique à usage topique compacte comprenant au moins un agent liant (AL).

15. Composition à usage topique (F) selon l'une des revendications 12 ou 13 comprenant une quantité efficace d'au moins un filtre organique solaire et/ou d'au moins un filtre inorganique solaire, pour protéger la peau humaine contre les effets inesthétiques sur ladite peau humaine des rayons ultra-violets du soleil.

16. Procédé de maquillage de la peau humaine **caractérisé en ce qu'**il comprend au moins une étape d'application sur ladite peau humaine d'une composition à usage topique (F) telle que définie à l'une ou quelconque des revendications 12 ou 13.

## Patentansprüche

1. Pulverförmige Zusammensetzung (C₁) in Form eines Puders, die pro 100 % ihrer Masse Folgendes umfasst:
i) 84,3 Massen-% bis 95,8 Massen-% mindestens eines Füllmittels (AC)
ii) 4 Massen-% bis 15 Massen-% mindestens eines Farbpigments (PC)
- iii) 0,2 Massen-% bis 0,7 Massen-% mindestens eines Polymers des Typs eines vernetzten anionischen Polyelektrolyts (P), das pro 100 Mol-% 65 Mol-% bis 95 Mol-% Monomereinheiten, die von partiell oder vollständig versalzter 2-Methyl-2-[(1-oxo-2-propenyl)amino]-1-propansulfonsäure stammen, 4,8 Mol-% bis 25 Mol-% Monomereinheiten, die von mindestens einem neutralen Monomer stammen, das aus den Elementen der Gruppe ausgewählt ist, bestehend aus 2-Hydroxyethylacrylat, 2-Hydroxyethylmethacrylat, N,N-Dialkylacrylamiden, wobei jede der Alkylgruppen zwischen einem und vier Kohlenstoffatomen umfasst, und 0,1 Mol-% bis 5 Mol-% Monomereinheiten, die von mindestens einem Monomer der Formel (I) stammen: 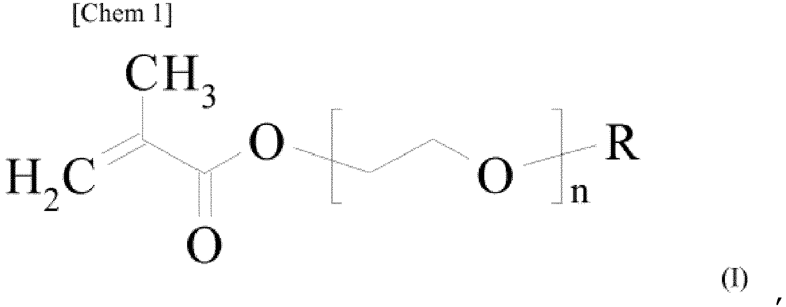 worin R einen geraden oder verzweigten Alkylrest mit acht bis zwanzig Kohlenstoffatomen darstellt und n eine Zahl größer als oder gleich null und kleiner als oder gleich zwanzig darstellt, und 0,1 Mol-% bis 5 Mol-% Monomereinheiten, die von mindestens einem diethylenischen oder polyethylenischen vernetzenden Monomer stammen, umfasst.

2. Pulverförmige Zusammensetzung (C₁) nach Anspruch 1, **dadurch gekennzeichnet, dass** in dem Polymer des Typs eines vernetzten anionischen Polyelektrolyts (P) das neutrale Monomer aus den Elementen der aus 2-Hydroxyethylacrylat oder N,N-Dimethylacrylamid bestehenden Gruppe ausgewählt ist.

3. Pulverförmige Zusammensetzung (C₁) nach einem der Ansprüche 1 oder 2, **dadurch gekennzeichnet, dass** in dem Polymer des Typs eines vernetzten anionischen Polyelektrolyts (P) das Monomer der Formel (I) aus den Elementen der Gruppe ausgewählt ist, bestehend aus tetraethoxyliertem Laurylmethacrylat der Formel (I₁), die der Formel (I) entspricht, in der R den n-Dodecyl-Rest darstellt und in der n gleich vier ist, aus Laurylmethacrylat der Formel (I₂), die Formel (I) entspricht, in der R einen n-Dodecyl-Rest darstellt und in der n gleich null ist, aus Isodecylmethacrylat der Formel (I'₂), die Formel (I) entspricht, in der R einen Isododecyl-Rest darstellt und in der n gleich Null ist, und aus Stearylmethacrylat der Formel (I₃), die Formel (I) entspricht, in der R einen n-Octadecyl-Rest darstellt und in der n gleich Null ist.

4. Pulverförmige Zusammensetzung (C₁) nach einem der Ansprüche 1 bis 3, **dadurch gekennzeichnet, dass** in dem Polymer des Typs eines vernetzten anionischen Polyelektrolyts (P) das diethylenische oder polyethylenische vernetzende Monomer aus Ethylenglykoldimethacrylat, Tetraallyloxyethan, Ethylenglykoldiacrylat, Diallylharnstoff, Triallylamin, Trimethylolpropantriacrylat, Methylenbis(acrylamid) oder einem Gemisch dieser Verbindungen ausgewählt ist.

5. Pulverförmige Zusammensetzung (C₁) nach einem der Ansprüche 1 bis 4, **dadurch gekennzeichnet, dass** das Polymer des Typs eines vernetzten anionischen Polyelektrolyts (P) ein mit Trimethylolpropantriacrylat vernetztes Polymer von partiell oder vollständig in Form des Ammoniumsalzes versalzter 2-Methyl-2-[(1-oxo-2-propenyl)amino]-1-propansulfonsäure, N,N-Dimethylacrylamid und tetraethoxyliertem Laurylmethacrylat der Formel (I₁) ist.

6. Pulverförmige Zusammensetzung (C₁) nach Anspruch 5, **dadurch gekennzeichnet, dass** das Polymer des Typs eines vernetzten anionischen Polyelektrolyts (P) pro 100 Mol-% Folgendes umfasst:
- 65 Mol-% bis 95 Mol-% Monomereinheiten, die von in der Ammoniumform versalzter 2-Methyl-2-[(1-oxo-2-propenyl)amino]-1-propansulfonsäure stammen;
- 4,8 Mol-% bis 25 Mol-% Monomereinheiten, die von N,N-Dimethylacrylamid stammen,
- 0,1 Mol-% bis 5 Mol-% Monomereinheiten, die von tetraethoxyliertem Laurylmethacrylat der Formel (I₁) stammen,
- 0,1 Mol-% bis 5 Mol-% Monomereinheiten, die von Trimethylolpropantriacrylat stammen.

7. Pulverförmige Zusammensetzung (C₁) nach einem der Ansprüche 1 bis 4, **dadurch gekennzeichnet, dass** das Polymer des Typs eines vernetzten anionischen Polyelektrolyts (P) ein Polymer von partiell oder vollständig in Form des Natriumsalzes versalzter 2-Methyl-2-[(1-oxo-2-propenyl)amino]-1-propansulfonsäure, 2-Hydroxyethylacrylat, Laurylmethacrylat der Formel (I₂) oder Isodecylmethacrylat der Formel (I'₂) und Stearylmethacrylat der Formel (I₃) in einem Molverhältnis von (I₂)/(I₃) oder (I'₂)/(I₃) von größer als oder gleich 1/10 und kleiner als oder gleich 10/1 ist und mit Trimethylolpropantriacrylat vernetzt ist.

8. Pulverförmige Zusammensetzung (C₁) nach Anspruch 7, **dadurch gekennzeichnet, dass** das Polymer des Typs eines vernetzten anionischen Polyelektrolyts (P) pro 100 Mol-% Folgendes umfasst:
- 70 Mol-% bis 95 Mol-% Monomereinheiten, die von in der Natriumform versalzter 2-Methyl-2-[(1-oxo-2-propenyl)amino]-1-propansulfonsäure stammen;
- 4,8 Mol-% bis 20 Mol-% Monomereinheiten, die von 2-Hydroxyethylacrylat stammen,
- 0,1 Mol-% bis 5 Mol-% Monomereinheiten, die von Laurylmethacrylat der Formel (I₂) oder Isodecylmethacrylat der Formel (I'₂) und Stearylmethacrylat der Formel (I₃) in einem Molverhältnis von (I₂)/(I₃) oder (I'₂)/(I₃) größer als oder gleich 1/6 und kleiner als oder gleich 6/1 stammen,
- 0,1 Mol-% bis 5 Mol-% Trimethylolpropantriacrylat.

9. Pulverförmige Zusammensetzung (C₁) nach einem der Ansprüche 1 bis 8, **dadurch gekennzeichnet, dass** das Füllmittel (AC) aus den Elementen der Gruppe ausgewählt ist, die aus Füllstoffen des lamellaren mineralischen Typs, Füllstoffen des lamellaren organischen Typs, Füllstoffen des sphärischen mineralischen Typs und Füllstoffen des sphärischen organischen Typs besteht.

10. Pulverförmige Zusammensetzung (C₁) nach einem der Ansprüche 1 bis 9, **dadurch gekennzeichnet, dass** das Farbpigment (PC) aus den Elementen der aus mineralischen Pigmenten, organischen Pigmenten und Perlglanzpigmenten bestehenden Gruppe ausgewählt ist.

11. Pulverförmige Zusammensetzung (C₁) nach einem der Ansprüche 1 bis 10, **dadurch gekennzeichnet, dass** sie pro 100 % ihrer Masse einen Massengehalt an Wasser von weniger als oder gleich 2 Massen-% aufweist.

12. Zusammensetzung zur topischen Anwendung (F), die pro 100 % ihrer Masse Folgendes umfasst:
i) 90 bis 97 Massen-% mindestens einer pulverförmigen Zusammensetzung (C₁) nach einem der Ansprüche 1 bis 11
ii) 3 bis 10 Massen-% mindestens eines bei 20°C flüssigen Fettbindemittels (AL).

13. Zusammensetzung zur topischen Anwendung (F) nach Anspruch 12, **dadurch gekennzeichnet, dass** das Bindemittel (AL) aus den Elementen der aus Rizinusöl, Octyldodecanol, Dimethicon, Cetearylethylhexanoat, Isopropylmyristat bestehenden Gruppe ausgewählt ist.

14. Verwendung eines Polymers des Typs eines vernetzten anionischen Polyelektrolyts (P) nach einem der Ansprüche 1 bis 8 zur Verbesserung der Schlagzähigkeit einer kompakten kosmetischen Formulierung zur topischen Anwendung, die mindestens ein Bindemittel (AL) umfasst.

15. Zusammensetzung zur topischen Anwendung (F) nach einem der Ansprüche 12 oder 13, die eine wirksame Menge mindestens eines organischen Sonnenschutzfilters und/oder mindestens eines anorganischen Sonnenschutzfilters zum Schützen der menschlichen Haut vor den unästhetischen Wirkungen ultravioletter Sonnenstrahlen auf die menschliche Haut umfasst.

16. Verfahren zum Schminken der menschlichen Haut, **dadurch gekennzeichnet, dass** es mindestens einen Schritt des Auftragens einer Zusammensetzung zur topischen Anwendung (F) nach einem der Ansprüche 12 oder 13 auf die menschliche Haut umfasst.

## Claims

1. Pulverulent composition (C₁) in the form of a powder comprising, per 100% of its mass:
i) from 84.3% to 95.8% by mass of at least one filling agent (FA)
ii) from 4% to 15% by mass of at least one colored pigment (CP)
- iii) from 0.2% to 0.7% by mass of at least one polymer of crosslinked anionic polyelectrolyte type (P) which comprises, per 100 mol%, from 65 mol% to 95 mol% of monomer units derived from partially or totally salified 2-methyl-2-[(1-oxo-2-propenyl)amino]-1-propanesulfonic acid, from 4.8 mol% to 25 mol% of monomer units derived from at least one neutral monomer chosen from the elements of the group consisting of 2-hydroxyethyl acrylate, 2-hydroxyethyl methacrylate, N,N-dialkylacrylamides in which each of the alkyl groups comprises between one and four carbon atoms, and from 0.1 mol% to 5 mol% of monomer units derived from at least one monomer of formula (I): 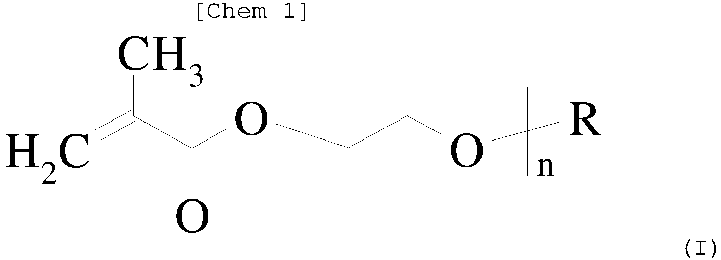 in which R represents a linear or branched alkyl radical comprising from eight to twenty carbon atoms and n represents a number of greater than or equal to zero and of less than or equal to twenty, and from 0.1 mol% to 5 mol% of monomer units derived from at least one diethylenic or polyethylenic crosslinking monomer.

2. Pulverulent composition (C₁) according to Claim 1, **characterized in that**, in the polymer of crosslinked anionic polyelectrolyte type (P), said neutral monomer is chosen from the elements of the group consisting of 2-hydroxyethyl acrylate or N,N-dimethylacrylamide.

3. Pulverulent composition (C₁) according to either of Claims 1 and 2, **characterized in that**, in the polymer of crosslinked anionic polyelectrolyte type (P), said monomer of formula (I) is chosen from the elements of the group consisting of tetraethoxylated lauryl methacrylate of formula (I₁), corresponding to formula (I) in which R represents an n-dodecyl radical and in which n is equal to four, of lauryl methacrylate of formula (I₂), corresponding to formula (I) in which R represents an n-dodecyl radical and in which n is equal to zero, of isodecyl methacrylate of formula (I'₂), corresponding to formula (I) in which R represents an isododecyl radical and in which n is equal to zero, and of stearyl methacrylate of formula (I₃), corresponding to formula (I) in which R represents an n-octadecyl radical and in which n is equal to zero.

4. Pulverulent composition (C₁) according to one of Claims 1 to 3, **characterized in that**, in the polymer of crosslinked anionic polyelectrolyte type (P), the diethylenic or polyethylenic crosslinking monomer is chosen from ethylene glycol dimethacrylate, tetraallyloxyethane, ethylene glycol diacrylate, diallylurea, triallylamine, trimethylolpropane triacrylate, methylenebis(acrylamide), or a mixture of these compounds.

5. Pulverulent composition (C₁) according to one of Claims 1 to 4, **characterized in that** said polymer of crosslinked anionic polyelectrolyte type (P) is a polymer of 2-methyl-2-[(1-oxo-2-propenyl)amino]-1-propanesulfonic acid, partially or totally salified in ammonium salt form, of N,N-dimethylacrylamide and of tetraethoxylated lauryl methacrylate of formula (I₁), crosslinked with trimethylolpropane triacrylate.

6. Pulverulent composition (C₁) according to Claim 5, **characterized in that** said polymer of crosslinked anionic polyelectrolyte type (P) comprises, per 100 mol%:
- from 65 mol% to 95 mol% of monomer units derived from 2-methyl-2-[(1-oxo-2-propenyl)amino]-1-propanesulfonic acid, salified in ammonium form;
- from 4.8 mol% to 25 mol% of monomer units derived from N,N-dimethylacrylamide,
- from 0.1 mol% to 5 mol% of monomer units derived from tetraethoxylated lauryl methacrylate of formula (I₁),
- from 0.1 mol% to 5 mol% of monomer units derived from trimethylolpropane triacrylate.

7. Pulverulent composition (C₁) as defined in one or any of Claims 1 to 4, **characterized in that** said polymer of crosslinked anionic polyelectrolyte type (P) is a polymer of 2-methyl-2-[(1-oxo-2-propenyl)amino]-1-propanesulfonic acid, partially or totally salified in sodium salt form, of 2-hydroxyethyl acrylate, of lauryl methacrylate of formula (I₂) or isodecyl methacrylate of formula (I'₂) and of stearyl methacrylate of formula (I₃), in a molar ratio of (I₂)/(I₃) or (I'₂)/ (I₃) of greater than or equal to 1/10 and less than or equal to 10/1, and crosslinked with trimethylolpropane triacrylate.

8. Pulverulent composition (C₁) as defined in Claim 7, **characterized in that** said polymer of crosslinked anionic polyelectrolyte type (P) comprises, per 100 mol%:
- from 70 mol% to 95 mol% of monomer units derived from 2-methyl-2-[(1-oxo-2-propenyl)amino]-1-propanesulfonic acid, salified in sodium form;
- from 4.8 mol% to 20 mol% of monomer units derived from 2-hydroxyethyl acrylate,
- from 0.1 mol% to 5 mol% of monomer units derived from lauryl methacrylate of formula (I₂) or isodecyl methacrylate of formula (I'₂), and from stearyl methacrylate of formula (I₃), in a molar ratio of (I₂)/(I₃) or (I'₂)/(I₃) of greater than or equal to 1/6 and less than or equal to 6/1,
- from 0.1 mol% to 5 mol% of trimethylolpropane triacrylate.

9. Pulverulent composition (C₁) as defined in one or any of Claims 1 to 8, **characterized in that** the filling agent (FA) is chosen from the elements of the group consisting of fillers of inorganic lamellar type, fillers of organic lamellar type, fillers of inorganic spherical type and fillers of organic spherical type.

10. Pulverulent composition (C₁) as defined in one or any of Claims 1 to 9, **characterized in that** the colored pigment (CP) is chosen from the elements of the group consisting of inorganic pigments, organic pigments and pearlescent pigments.

11. Pulverulent composition (C₁) as defined in one or any of Claims 1 to 10, **characterized in that** it comprises, per 100% of its mass, a content by mass of water of less than or equal to 2% by mass.

12. Composition for topical use (F) comprising, per 100% of its mass:
i) from 90% to 97% by mass of at least one pulverulent composition (C₁) as defined in one or any of Claims 1 to 11
ii) from 3% to 10% by mass of at least one fatty binding agent (BA) liquid at 20°C.

13. Composition for topical use (F) as defined in Claim 12, **characterized in that** the binding agent (BA) is selected from the elements of the group consisting of castor oil, octyldodecanol, dimethicone, cetearyl ethylhexanoate, and isopropyl myristate.

14. Use of a polymer of crosslinked anionic polyelectrolyte type (P) as defined in one or any of Claims 1 to 8 for improving the impact strength of a compact cosmetic formulation for topical use comprising at least one binding agent (BA).

15. Composition for topical use (F) according to either of Claims 12 and 13, comprising an effective amount of at least one organic sunscreen and/or at least one inorganic sunscreen, for protecting human skin against the unaesthetic effects on said human skin of the ultraviolet rays of the sun.

16. Process for making up human skin, **characterized in that** it comprises at least one step of applying to said human skin a composition for topical use (F) as defined in one or either of Claims 12 and 13.
